# EUROPEAN PATENT SPECIFICATION

(11) **EP 4 244 227 B1**
(45) Date of publication and mention of the grant of the patent: **22.04.2026**
(21) Application number: 21890406.8
(22) Date of filing: 12.11.2021
(51) Int. Cl.: C07D 491/107, G01N 21/64, G01N 33/52, C07C 211/31, C07C 309/43, C07C 327/18, C07D 215/40, C07D 231/50, C07D 239/48, C07D 311/02, C07D 455/04, C07D 491/20, C07D 517/20, G01N 33/58, G01N 33/84, G01N 33/94, C09B 11/12, C09B 67/22

(54) **FLUORESCENT SENSORS**
FLUORESZIERENDE SENSOREN
CAPTEURS FLUORESCENTS

(30) Priority: 13.11.2020 AU 2020904179
(43) Date of publication of application: 20.09.2023
(73) Proprietor: The University Of Sydney, New South Wales 2006 (AU)
(72) Inventor: NEW, Elizabeth, Sydney, New South Wales 2006 (AU); MITCHELL, Linda, Sydney, New South Wales 2006 (AU); SHEN, Clara, Sydney, New South Wales 2006 (AU)
(74) Representative: Mewburn Ellis LLP
(86) International application number: PCT/AU2021/051345
(87) International publication number: WO 2022/099374

(56) References cited:
- CN-A- 111 875 611
- CN-A- 111 875 611
- KOLANOWSKI JACEK L ET AL: "A fluorescent probe for investigating metabolic stability of active transplatin analogues", SENSORS AND ACTUATORS B: CHEMICAL, vol. 255, 14 September 2017 (2017-09-14), pages 2721 - 2724, XP085259552, ISSN: 0925-4005, DOI: 10.1016/J.SNB.2017.09.084
- SHEN CLARA ET AL: "Fluorescent sensing of monofunctional platinum species", vol. 51, no. 29, 1 January 2015 (2015-01-01), UK, pages 6312 - 6314, XP055938478, ISSN: 1359-7345, Retrieved from the Internet <URL:https://pubs.rsc.org/en/content/articlepdf/2015/cc/c4cc08077g> DOI: 10.1039/C4CC08077G
- SHEN CLARA, HARRIS BENJAMIN D. W., DAWSON LUCY J., CHARLES KELLIE A., HAMBLEY TREVOR W., NEW ELIZABETH J.: "Fluorescent sensing of monofunctional platinum species", CHEMICAL COMMUNICATIONS, ROYAL SOCIETY OF CHEMISTRY, UK, vol. 51, no. 29, 1 January 2015 (2015-01-01), UK , pages 6312 - 6314, XP055938478, ISSN: 1359-7345, DOI: 10.1039/C4CC08077G
- ONG, JX ET AL.: "Structure-activity relationship studies on rhodamine B-based fluorogenic probes and their activation by anticancer platinum (II) compounds", JOURNAL OF INORGANIC BIOCHEMISTRY, vol. 153, 2015, pages 272 - 278, XP029350257, DOI: 10.1016/j.jinorgbio.2015.10.002
- KOLANOWSKI, JL ET AL.: "A fluorescent probe for investigating metabolic stability of active transplatin analogues", SENSORS AND ACTUATORS B: CHEMICAL, vol. 255, 2018, pages 2721 - 2724, XP085259552, DOI: 10.1016/j.snb.2017.09.084

## Description

### Field of the invention

The present invention relates to a novel fluorescent sensor for detecting platinum complexes in test samples, combinations comprising same, and methods comprising use of same.

### Background of the invention

Platinum and platinum complexes are important chemicals in medicine, industry and environmental contamination.

For example, platinum-based drugs are currently one of the most widely-used classes of cancer chemotherapeutics. The three complexes that have approval worldwide: cisplatin, oxaliplatin and carboplatin, are used either alone or in combination for treating most cancer types, particularly solid tumours. Whilst effective at treating cancer, the side-effects accompanying platinum-based chemotherapy are severe and create challenges during treatment. In particular, major side-effects such as nephrotoxicity, myelosuppression and neurotoxicity are often severe enough to necessitate dose-reduction, reducing the efficacy of treatment. Directly monitoring platinum levels in patients during treatment, rather than monitoring the resulting toxicity, could enable more effective modification of dosages and overall treatment plans.

Quantification of platinum levels in human samples has traditionally been achieved by graphite furnace atomic absorbance spectroscopy (GF-AAS). This has more recently been superseded by inductively coupled plasma mass spectrometry (ICP-MS), which has a lower limit of detection. Whilst an extremely sensitive and reliable technique, ICP-MS only provides information about total platinum concentration and cannot report on oxidation state or coordination environment. Sensitivity to these features are crucial for understanding speciation and distinguishing active drug from protein-bound, and largely inactive, platinum species. Other techniques such as nuclear magnetic resonance (NMR), high-performance liquid chromatography (HPLC) and X-ray absorbance near edge structure (XANES) can provide structural information, but do this at the cost of sensitivity or instrument accessibility.

Fluorescent sensing is an emerging tool to study metals in biology due to its high sensitivity and relatively accessible instrumentation. There are a small number of reported fluorescent sensors for studying platinum complexes. However, these sensors have limited application. The sensors have typically been designed for high specificity towards one analyte, limiting the number of analytes they are able to detect. Further, the growing number of platinum complexes being developed as potential drug candidates makes achieving selectivity for each complex an ongoing challenge. An added difficulty is achieving high selectivity in complex biological matrices such as blood.

Therefore, there is a need for alternative sensors and methods for detecting platinum complexes in samples, including complex samples. CN 111875611 A discloses for example compounds useful for the detection of anti-cancer platinum prodrugs. Fluorescent sensors for detecting platinum have also been reported by Kolanowski et al, Sensors and Actuators B: Chemical, 2018, 2721 and Shen et al, Chem. Commun., 2015, 6312.

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

### Summary of the invention

The present invention is predicated at least in part on the discovery that a compound of formula I is capable of acting as a fluorogenic sensor for metal complexes, including platinum complexes, and may be useful for detecting platinum complexes in samples, including complex samples.

Accordingly, the present invention provides a compound of formula I: 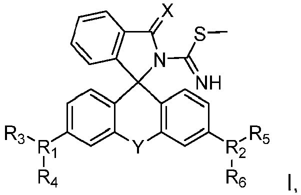 wherein:
X is O or S;
Y is selected from O, Si, Se and Ge;
R₁ and R₂ are independently N or O; and
R₃-R₆ are independently absent or H or C₁-C₆alkyl,
or a salt or solvate thereof.

In preferred embodiments, the compound of formula I is a compound of formula Ia: 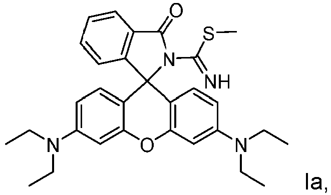 or a salt or solvate thereof.

The compound of formula I may be used in combination with one or more other fluorogenic sensors. Accordingly, the present invention also provides a combination comprising a compound of formula I, or a salt or solvate thereof, preferably of formula la, one or more additional fluorogenic sensors. Preferably, the one or more additional fluorogenic sensors are selected from those described herein.

Accordingly, the present invention also provides a combination comprising:
- the compound of formula I, or a salt or a solvate thereof; and
- one or more compounds which are fluorogenic sensors selected from:
   a compound of formula II: 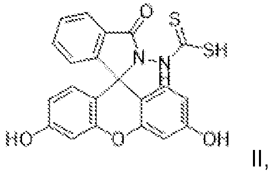
   a compound of formula III: 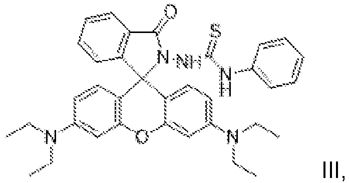
   a compound of formula IV: 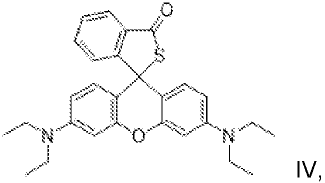
   a compound of formula V: 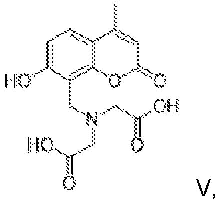
   a compound of formula VI: 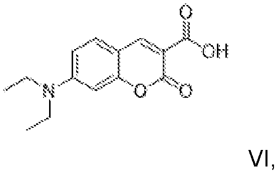
   a compound of formula VIII: 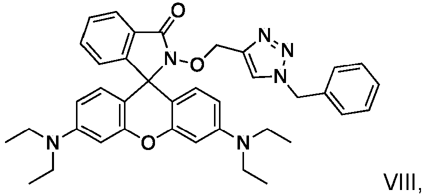
   a compound of formula IX: 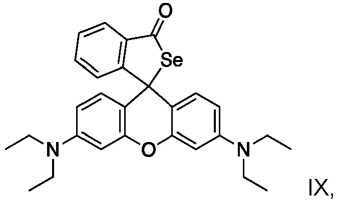
   a compound of formula XI: 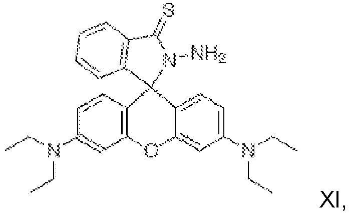
   a compound of formula XII: 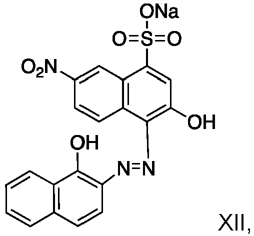
   a compound of formula XIII: 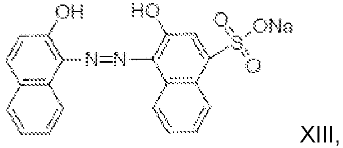
   a compound of formula XVIII: 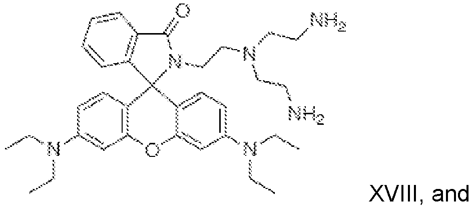
   a compound of formula XIX: 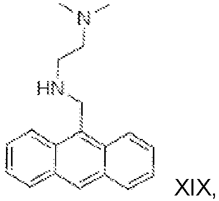
   or salts or solvates thereof.

In some embodiments, the combination comprises the compound of formula I, preferably la, and one or more compounds selected from the compounds of formulae II-VI, VIII, XII and XIII, or salts or solvates thereof.

In some embodiments, the combination comprises the compound of formula I, preferably la, and one or more compounds selected from the compounds of formulae II-VI, IX, XI, XVIII and XIX, or salts or solvates thereof.

In preferred embodiments, the combination comprises the compound of formula I and the compounds of formulae II-VI, or salts or solvates thereof.

The present invention also provides the use of the compound of formula I, preferably la, or a salt or a solvate thereof, or the use of the combination of the compound of formula I, preferably la, and one or more of the compounds of formulae II-VI, VIII, IX, XI-XIII, XVIII and XIX, or salts or solvates thereof, for detecting one or more platinum complexes in a test sample. Preferably the use involves the compounds of formulae I-VI for detecting platinum complexes in a test sample.

The present invention also provides a method for detecting a platinum complex in a test sample, the method comprising:
- contacting a test sample suspected of comprising a platinum complex with the compound of formula I, or a salt or a solvate thereof, or the combination of the compound of formula I, preferably la, and one or more of the compounds of formulae II-VI, VIII, IX, XI-XIII, XVIII and XIX, or salts or solvates thereof;
- exposing the test sample to a source of light sufficient to excite the compound of formula I, preferably la, or the combination of compounds; and
- detecting fluorescence from the compound of formula I or the combination of compounds, wherein the presence of fluorescence indicates the presence of a platinum complex in the test sample.

The present invention also provides a method for identifying a platinum complex in a test sample, the method comprising:
- providing a test sample suspected of comprising a platinum complex;
- contacting the test sample with the combination of the compound of formula I, preferably la, and one or more of the compounds of formulae II-VI, VIII, IX,XI-XIII, XVIII and XIX, or salts or solvates thereof;
- exposing the test sample to a source of light sufficient to excite the combination of compounds; and
- detecting a fluorescence response pattern from the combination of compounds;
- comparing the fluorescence response pattern of the test sample with a reference data set in the form of fluorescence response patterns from one or more reference samples each comprising a metal ion complex to identify whether there is a difference in the fluorescence response pattern of the test sample and the one or more reference samples; and
determining the identity of the platinum complex in the test sample.

The present invention also provides a method for determining the concentration of a platinum complex in a test sample, the method comprising:
- providing a test sample comprising the platinum complex;
- contacting the test sample with the combination of the compound of formula I, preferably la, and one or more of the compounds of formulae II-VI, VIII, IX, XI-XIII, XVIII and XIX, or salts or solvates thereof,
- exposing the test sample to a source of light sufficient to excite the combination of compounds;
- detecting a fluorescence response pattern from the combination of compounds;
- comparing the fluorescence response pattern of the test sample with a reference data set in the form of fluorescence response patterns from one or more reference samples each comprising a concentration of the platinum complex to identify whether there is a difference in the fluorescence response pattern as between the test sample and the one or more reference samples; and
- determining the concentration of the platinum complex in the test sample, where the fluorescence response pattern of the platinum complex in the test sample is the same as the fluorescence response pattern of the reference sample having the same concentration.

The present invention also provides a method for monitoring the concentration of a platinum-containing drug in a subject who is receiving a treatment regimen comprising the platinum-containing drug, the method comprising:
- providing a biological sample obtained from the subject during the treatment regimen; and
- determining the concentration of the platinum-containing drug in the biological sample according to the method for determining the concentration of a platinum complex described herein.

The present invention also provides a kit comprising the compound of formula I, preferably la, or a salt or solvate thereof, or the combination of the compound of formula I, preferably la, and one or more of the compounds of formulae II-VI, VIII, IX, XI-XIII, XVIII and XIX, or salts or solvates thereof. Preferably the kit is for use in accordance with a method described herein. Optionally the kit also comprises written instructions for utilising the compound, or combination of compounds, as described herein.

As used herein, except where the context requires otherwise, the term "comprise" and variations of the term, such as "comprising", "comprises" and "comprised", are not intended to exclude further additives, components, integers or steps.

Further aspects of the present invention and further embodiments of the aspects described in the preceding paragraphs will become apparent from the following description, given by way of example and with reference to the accompanying drawings.

### Brief description of the drawings

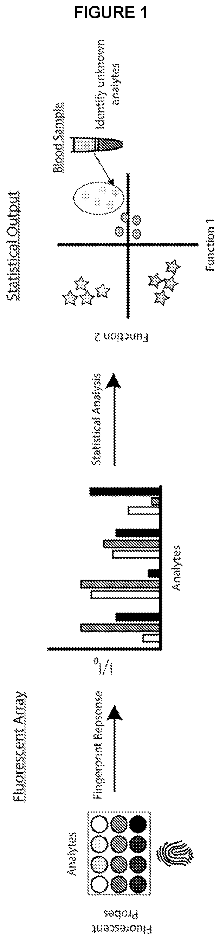

**Figure 1****.** Schematic showing the conversion of the fluorescence signal measured by the fluorescent sensor array into the characterisation of the analyte.

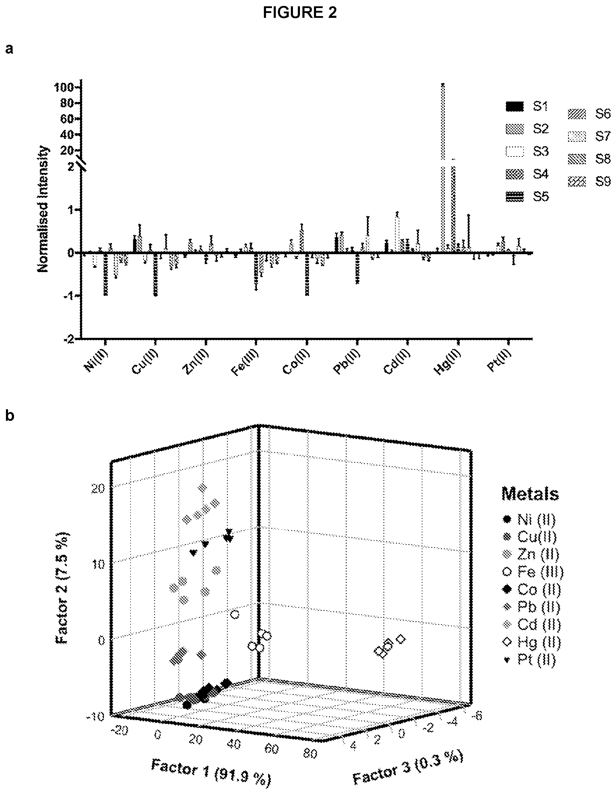

**Figure 2****.** Fluorescence response pattern (I/I₀ - 1) of the fluorescence sensor array using sensor molecules S1-S9 (10 µM) with 9 metal ions (50 µM) in DMF (2% v/v) diluted in HEPES buffer (20 mM, pH 7.4), presented as a) normalised intensity, and b) three-dimensional LDA score plot of the first three factors obtained. Sensors S1-S9 in Figure 2a are depicted as follows: S1 - black bars; S2 - grey bars; S3 - white bars; S4 - checkered bars; S5 - horizontal striped bars; S6 - right diagonal striped bars; S7 - dotted bars; S8 - left diagonal striped bars; S9 - brick pattern bars. Metal ions in Figure 2b are depicted as follows: Ni(ll) - black circles; Cu(ll) - dark grey circles; Zn(ll) - light grey circles; Fe(lll) - white circles; Co(ll) - black diamonds; Pb(ll) - dark grey diamonds; Cd(ll) - light grey diamonds; Hg(ll) - white diamonds; Pt(ll) - black triangles.

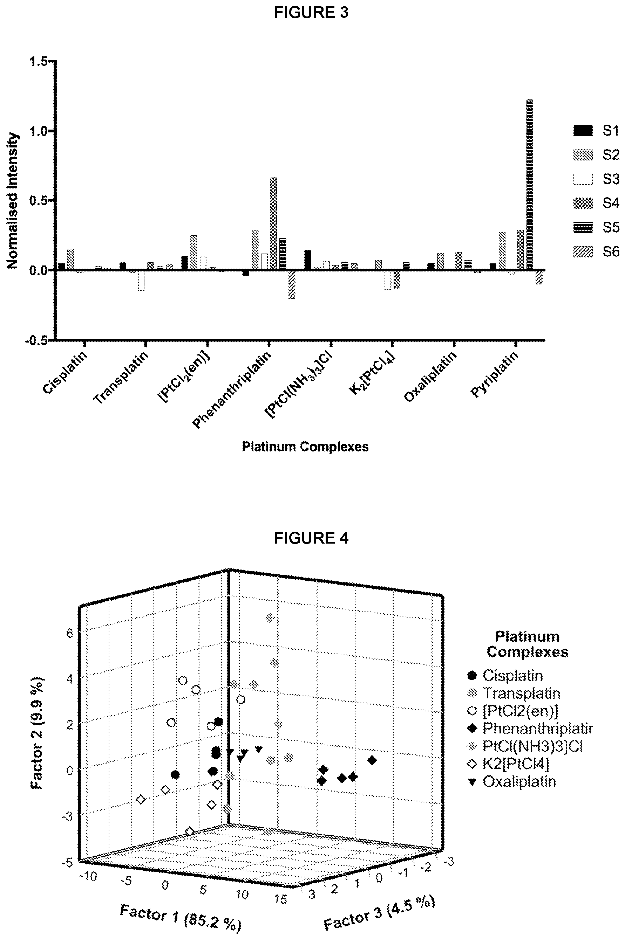

**Figure 3****.** Fluorescence response pattern (I/I₀ - 1) of S1-S6 (200 µM) with 8 platinum complexes (200 µM) in DMF (2% v/v) diluted in HEPES buffer (20 nM, pH 7.4). Sensors S1-S6 in Figure 3 are depicted as follows: S1 - black bars; S2 - grey bars; S3 - white bars; S4 - checkered bars; S5 - horizontal striped bars; S6 - right diagonal striped bars.
**Figure 4****.** Three-dimensional score plot of the first three factors obtained from LDA of the fluorescence response pattern (I/I₀ - 1) of S1-S6 (200 µM) with 7 platinum complexes (200 µM) in DMF (2% v/v) diluted in HEPES buffer (20 mM, pH 7.4). Platinum complexes in Figure 4 are depicted as follows: cisplatin - black circles; transplatin - grey circles; [PtCl₂(en)] - white circles; phenanthriplatin - black diamonds; [PtCl(NH₃)₃]Cl - grey diamonds; K₂[PtCl₄] - white diamonds; oxaliplatin - black triangles.

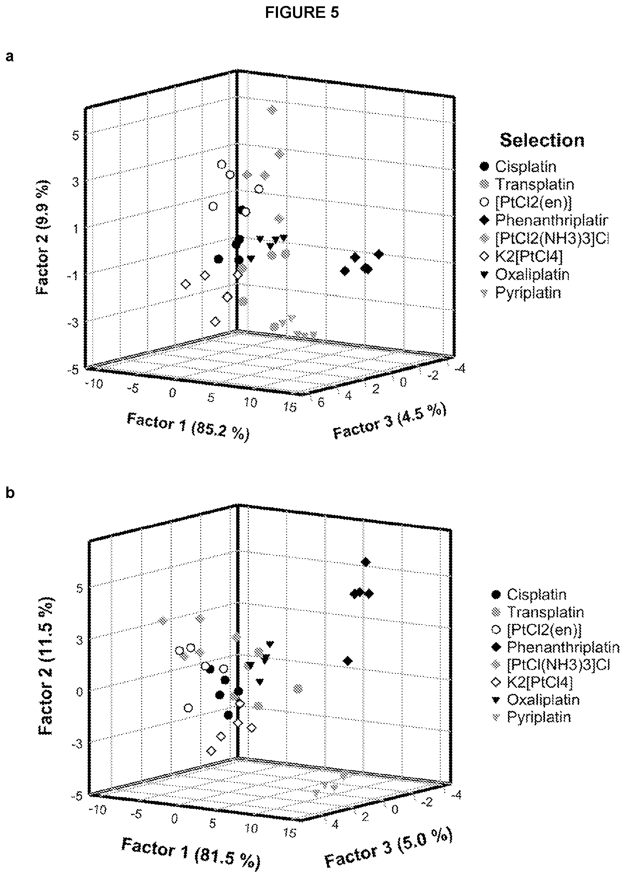

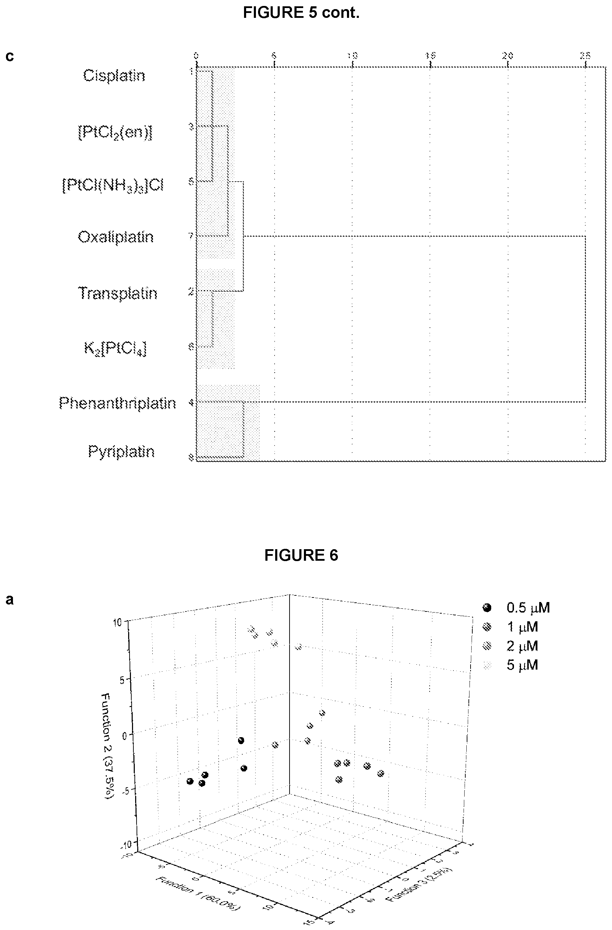

**Figure 5****.** Three-dimensional score plot of the first three factors obtained from LDA of the fluorescence response pattern (I/I₀ - 1) of S1-S6 (200 µM) in DMF (2% v/v) diluted in HEPES buffer (20 mM, pH 7.4) and a) 7 platinum complexes and pyriplatin as an unknown class, and b) 7 platinum complexes and pyriplatin as an eighth class. Fluorescence response pattern (I/I₀ - 1) of S1-S6 (200 µM) towards 8 platinum complexes in DMF (2% v/v) diluted in HEPES buffer (20 mM, pH 7.4), presented at c) HAD dendrogram. Grey areas in Figure 5c represent the three major clusters corresponding to different platinum configurations. Platinum complexes in Figures 5a and 5b are depicted as follows: cisplatin - black circles; transplatin - grey circles; [PtCl₂(en)] - white circles; phenanthriplatin - black diamonds; [PtCl(NH₃)₃]Cl - grey diamonds; K₂[PtCl₄] - white diamonds; oxaliplatin - black triangles; pyriplatin - grey triangles.

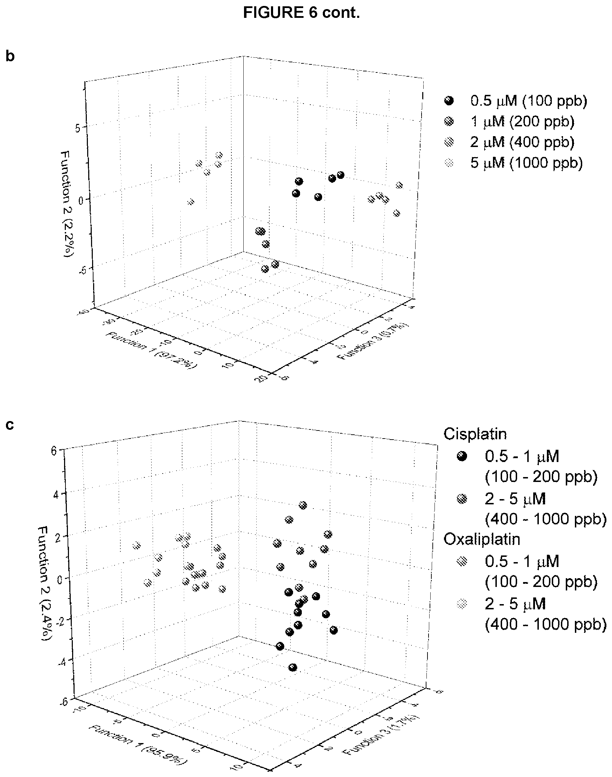

**Figure 6****.** Three-dimensional score plot of the first three factors obtained from LDA of the fluorescence response pattern (I/I₀ - 1) of S1-S6 (10 µM) in DMF (4% v/v) diluted in PBS buffer (20 mM, pH 7.4, 46% v/v) and human plasma (50% v/v) spiked with a) cisplatin (0.5-5 µM), b) oxaliplatin (0.5-5 µM) or c) both cisplatin and oxaliplatin (each 0.5-5 µM). Concentrations of cisplatin and oxaliplatin in Figures 6a and 6b respectively are depicted as follows: 0.5 µM (100 ppb) - black spheres; 1 µM (200 ppb) - dark grey spheres; 2 µM (400 ppb) - light grey spheres; 5 µM (1000 ppb) - white spheres. Concentrations of cisplatin and oxaliplatin in Figure 6c are depicted as follows: cisplatin 0.5-1 µM (100-200 ppb) - black spheres; cisplatin 2-5 µM (400-1000 ppb) - dark grey spheres; oxaliplatin 0.5-1 µM (100-200 ppb) - light grey spheres; oxaliplatin 2-5 µM (400-1000 ppb) - white spheres.

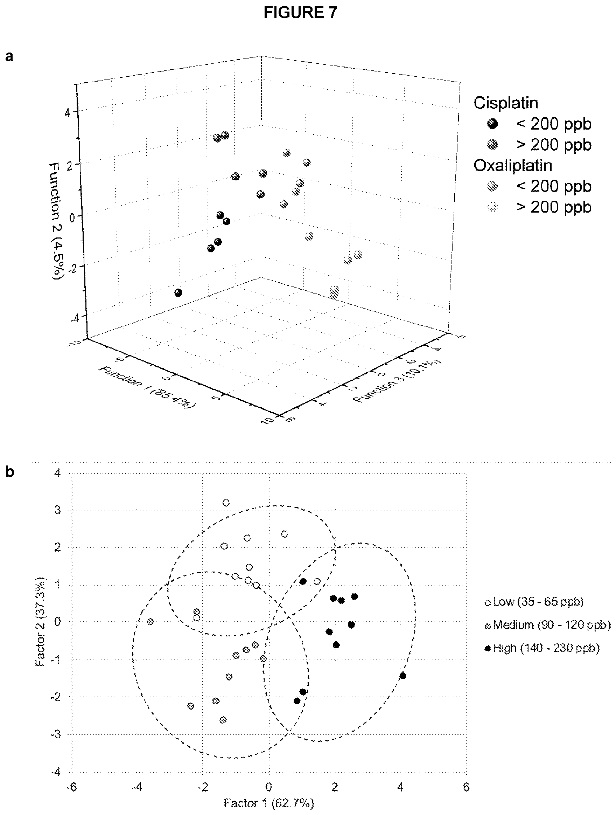

**Figure 7****.** Fluorescence response pattern (I/I₀ - 1) of S1-6 (10 µM) with clinical cisplatin and oxaliplatin samples in 20 mM PBS buffer at pH 7.4, presented as a) a three-dimensional LDA score plot of the first three factors obtained and b) a two-dimensional LDA score plot of the first two factors obtained with 95% confidence ellipses (dashed). Concentrations of cisplatin and oxaliplatin in Figure 7a are depicted as follows: cisplatin < 200 ppb - black spheres; cisplatin > 200 ppb - dark grey spheres; oxaliplatin < 200 ppb - light grey spheres; oxaliplatin > 200 ppb - white spheres. Concentrations in Figure 7b are depicted as follows: low (35-65 ppb) - white circles; medium (90-120 ppb) - grey circles; high (140-230 ppb) - black circles.

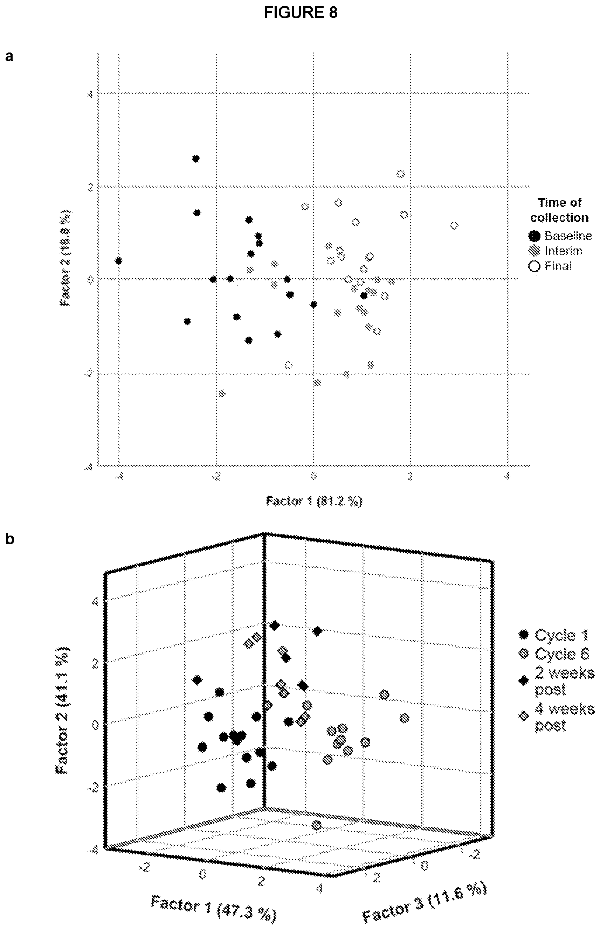

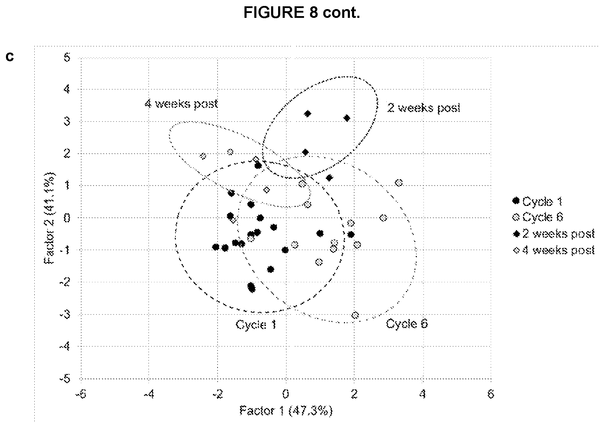

**Figure 8**. Fluorescence response pattern (I/I₀ - 1) of S1-6 (10 µM) with clinical oxaliplatin in 20 mM PBS buffer on 51 samples collected from the same 17 patients, collected at baseline, interim and final time points and presented as a) a two-dimensional LDA score plot of the first two factors obtained, and collected at treatment cycle 1, treatment cycle 6, 2 weeks post-treatment and 4 weeks post-treatment and presented as b) a three-dimensional LDA score plot of the first three factors obtained and c) a two-dimensional LDA score plot of the first two factors obtained with 95% confidence ellipses (dashed). Samples in Figure 8a are depicted as follows: baseline - black circles; interim - grey circles; final - white circles. Samples in Figures 8b and 8c are represented as follows: cycle 1 - black circles; cycle 6 - grey circles; 2 weeks post treatment - black diamonds; 4 weeks post treatment - grey diamonds.

### Detailed description of the embodiments

The present invention provides a compound of formula I: 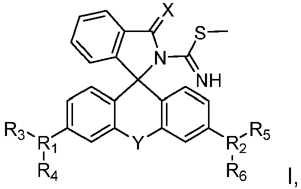 wherein:
X is O or S;
Y is selected from O, Si, Se and Ge;
R₁ and R₂ are independently N or O; and
R₃-R₆ are independently absent or H or C₁-C₆alkyl,
or a salt or a solvate thereof.

In some embodiments of formula I, one or more of the following applies:
X is O;
Y is O;
R₁ and/or R₂ are N;
R₃ and/or R₄ and/or R₅ and/or R₆ are ethyl.

In preferred embodiments, the compound of formula I is a compound of formula la: 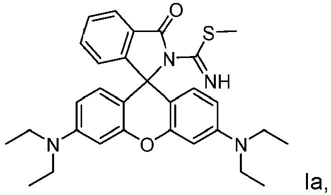 or a salt or solvate thereof.

The term "alkyl" as used herein refers to straight chain or branched hydrocarbon groups. Where appropriate, the alkyl group may have a specified number of carbon atoms, for example, C₁₋₆alkyl which includes alkyl groups having 1, 2, 3, 4, 5 or 6 carbon atoms in a linear or branched arrangement. Examples of suitable alkyl groups include, but are not limited to, methyl, ethyl, n-propyl, i-propyl, n-butyl, i-butyl, t-butyl, n-pentyl, 2-methylbutyl, 3-methylbutyl, 4-methylbutyl, n-hexyl, 2-methylpentyl, 3-methylpentyl, 4-methylpentyl, 5-methylpentyl, 2-ethylbutyl and 3-ethylbutyl.

Suitable salts include, but are not limited to, salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, malic, citric, lactic, mucic, gluconic, benzoic, succinic, oxalic, phenylacetic, methanesulphonic, toluenesulphonic, benzenesulphonic, salicylic, sulphanilic, aspartic, glutamic, edetic, stearic, palmitic, oleic, lauric, pantothenic, tannic, ascorbic and valeric acids.

Base salts include, but are not limited to: those formed with pharmaceutically acceptable cations, such as: sodium, potassium, lithium, calcium, magnesium, zinc, ammonium and alkylammonium; salts formed from triethylamine; alkoxyammonium salts such as those formed with ethanolamine; and salts formed from ethylenediamine, choline or amino acids such as arginine, lysine or histidine.

Basic nitrogen-containing groups may be quarternised with such agents as lower alkyl halide, such as methyl, ethyl, propyl, and butyl chlorides, bromides and iodides; dialkyl sulfates like dimethyl and diethyl sulfate; and others.

Salts, or other derivatives of the compound of formula I and other compounds of the present invention may be provided in the form of solvates. Solvates contain either stoichiometric or non-stoichiometric amounts of a solvent, and may be formed during the process of crystallization with pharmaceutically acceptable solvents such as water, alcohols such as methanol, ethanol or isopropyl alcohol, DMSO, acetonitrile, dimethyl formamide (DMF) and the like with the solvate forming part of the crystal lattice by either non-covalent binding or by occupying a hole in the crystal lattice. Hydrates are formed when the solvent is water, alcoholates are formed when the solvent is alcohol. Solvates of the compounds of the present invention can be conveniently prepared or formed during the processes described herein. In general, the solvated forms are considered equivalent to the unsolvated forms for the purposes of the compounds and methods provided herein.

As used herein, the term "fluorophore" refers to a chemical moiety that emits light following excitation. Any suitable fluorophore may be used. Suitable fluorophores include, but are not limited to rhodamine and derivatives thereof, such as rhodamine B, rhodamine 6G, rhodamine 123, tetramethylrhodamine and silicon rhodamine, fluorescein and derivatives thereof, and coumarin and derivatives thereof.

The compound of formula I is a derivative of rhodamine B and exhibits fluorescence in the visible range between 400-600 nm. Advantageously, as shown in the Examples, the compound of formula I is also cross-reactive with different metal complexes and is sensitive to different coordination spheres of metal ions including platinum. Therefore, the compound of formula I may be useful as a fluorogenic sensor for detecting a platinum complex.

The compound of formula I, preferably la, may be used in combination with one or more other compounds which are fluorogenic sensors. It will be appreciated that the one or more fluorogenic sensors may be any fluorogenic sensor, preferably those which exhibit cross-reactivity with different metal complexes and are sensitive to different coordination spheres of metal ions, including those known in the art. In some embodiments, the one or more other fluorogenic sensors are selected from the following compounds, or salts or solvates thereof:

| **Cpd** | **Structure** | **Cpd** | **Structure** |
|---|---|---|---|
| II | 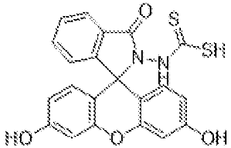 | III | 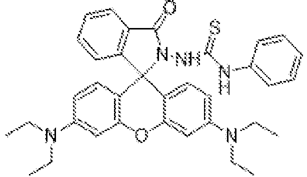 |
| IV | 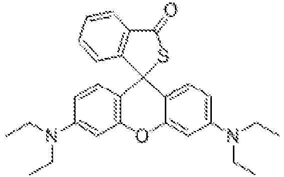 | V | 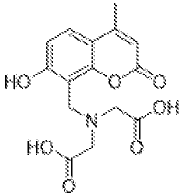 |
| VI | 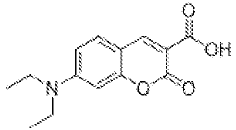 | VII | 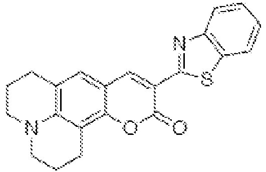 |
| VIII | 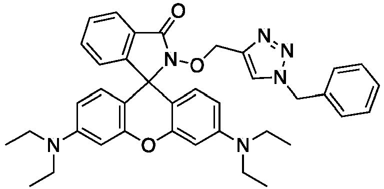 | IX | 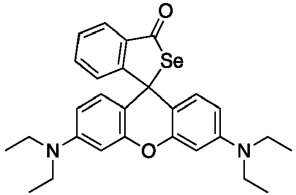 |
| X | 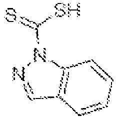 | XI | 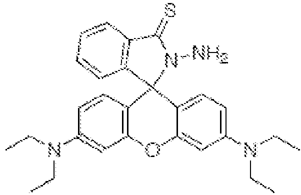 |
| XII | 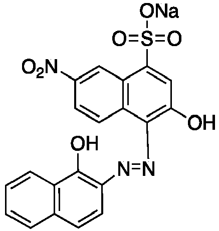 | XIII | 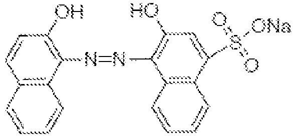 |
| XIV | 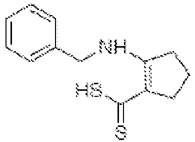 | XV | 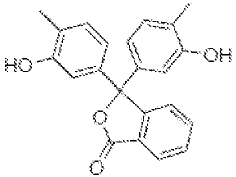 |
| XVI | 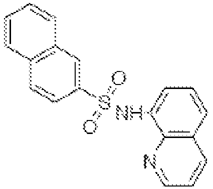 | XVII | 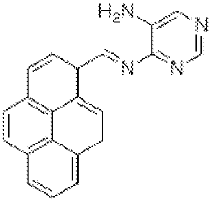 |
| XVIII | 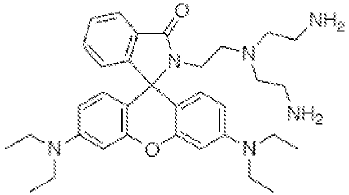 | XIX | 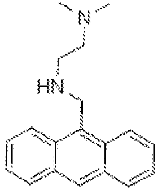 |
| XX | 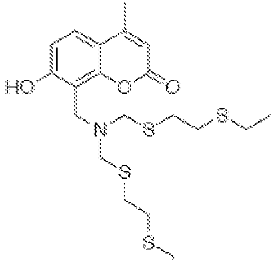 | | |

The compound of formula I, preferably of formula la, may be used in combination with two or more, three or more, four or more, five or more, six or more, seven or more, eight or more, or nine or more of the other fluorogenic sensors. In some embodiments, the compound of formula I, preferably la, may be used in combination with one, two, three, four, five, six, seven, eight, nine, ten, eleven, twelve, thirteen, fourteen, fifteen, sixteen, seventeen, eighteen, or all of the other fluorogenic sensors.

Accordingly, the present invention provides a combination comprising:
- the compound of formula I, preferably la, or a salt or solvate thereof; and
- one or more compounds selected from the compounds of formulae II-VI, VIII, IX, XI-XIII, XVIII and XIX, or salts or solvates thereof.

In some embodiments, the combination comprises the compound of formula I and two or more, three or more, four or more, five or more, six or more, seven or more, eight or more, nine or more, ten or more, or all eleven of the compounds of formulae II-VI, VIII, IX, XI-XIII, XVIII and XIX. In some embodiments, the combination comprises the compound of formula I, the compound of formula II, the compound of formula III, one of the compounds of formulae IV, IV and XI, two of the compounds of formulae V, VI, XVIII and XIX, the compound of formula VIII, the compound of formula XII, and the compound of formula XIII. In some embodiments, the combination comprises the compound of formula I, the compound of formula II, the compound of formula III, the compound of formula IV, the compound of formula V, the compound of formula VI, the compound of formula VIII, the compound of formula XII, and the compound of formula XIII. In some embodiments, the combination comprises the compound of formula I, the compound of formula II, the compound of formula III, one of the compounds of formulae IV, IV and XI, and two of the compounds of formulae V, VI, XVIII and XIX.

In some embodiments, the combination comprises the compound of formula I and one or more compounds selected from the compounds of formulae II-VI. In some embodiments, the combination comprises the compound of formula I and two or more, three or more, four or more, or all five of the compounds of formulae II-VI.

In some embodiments, the combination further comprises one or more compounds selected from the compounds of formulae VII, X, XIV-XVII and XX, or salts or solvates thereof.

In some embodiments, the combination comprises the compound of formula I and the compounds of formulae II-VI, VIII, XII and XIII, especially the compound of formula IA and the compounds of formulae II-VI, VIII, XII and XIII, or salts or solvates thereof. In some embodiments, the combination consists essentially of the compounds of formulae I-VI, VIII, XII and XIII, especially the compound of formula IA and the compounds of formulae II-VI, VIII, XII and XIII, or salts or solvates thereof. In this context, the term "consisting essentially of" means the combination includes only formulae I-VI, VIII, XII and XIII, or salts or solvates thereof, as the fluorogenic sensors in the combination. The compounds of formula IA and formulae II-VI, VIII, XII and XIII, are also referred to herein as sensors S3, S1, S2 and S4-S9, respectively.

In preferred embodiments, the combination comprises the compound of formula I and the compounds of formulae II-VI, especially the compound of formula IA and the compounds of formulae II-VI, or salts or solvates thereof. In some embodiments, the combination consists essentially of the compounds of formulae I-VI, especially the compound of formula IA and the compounds of formulae II-VI, or salts or solvates thereof. In this context, the term "consisting essentially of" means the combination includes only formulae I-VI, or salts or solvates thereof, as the fluorogenic sensors in the combination. The compounds of formula IA and formulae II-VI are also referred to herein as sensors S3, S1, S2, S4, S5 and S6, respectively.

In the methods and uses of the compound of formula I and the combination described herein, each compound may be used in an amount sufficient for the fluorescence of that compound to be detected. The amount of each compound may be suitably selected depending on the amount of metal ion complex(es) present in a sample to be analysed, if known. In some embodiments, the compound of formula I is present in an amount of from about 0.1 µM to about 10 µM. In some embodiments, each compound of the combination is independently present in an amount of from about 0.1 µM to about 10 µM.

Sensors S1-S9 exhibit fluorescence in the visible range between 350-600 nm: as shown in the Examples, S1 has a maximum excitation wavelength of 480 nm, S2-4 have a maximum excitation wavelength of 545 nm, S5 has a maximum excitation wavelength of 380 nm, S6-S7 have a maximum excitation wavelength of 445 nm and S8-S9 have a maximum excitation wavelength of 280 nm. Further, as shown in the Examples, sensors S1-S6 are sensitive to different coordination spheres of various metal ions. Sensors S1-S4 are based on rhodamine and fluorescein fluorophores, which have been modified with a spirolactam motif that is responsible for the turn-on/off behaviour of the fluorophores. Reaction-based ring opening of the spirolactam ring allows for transition from the off-to-on fluorescent state. The ring opening is enhanced by platinum complexes when soft atoms such as sulphur present in the binding pocket and there is an optimal binding site size. Therefore, sensors S1-S4 generate an enhanced fluorescence signal in the presence of almost all platinum complexes. The fluorescence signal is also influenced by the coordination sphere, ligand lability and size of the complex. Sensors S5 and S6 are coumarin derivatives that are fluorescent due to a pi-system with an electron-withdrawing lactone which can undergo an internal charge transfer (ICT) mechanism. Substituents on the aromatic system can either increase (e⁻donating) or decrease (e⁻ withdrawing) this process and consequently enhance or quench the fluorescence. Interactions with these substituents can also impact the fluorescence of the coumarin fluorophore. Advantageously, as shown in the Examples, sensors S1-S6 are capable of cross-reacting with various metal ions, including platinum and other metal ions from biological, environmental and industrial sources, and can generate unique fluorescent responses or "fingerprints" for each metal ion analyte. Therefore, compounds of the combination may be useful as fluorescent sensors or probes for detecting, identifying or quantifying a platinum complex in a test sample, for example as illustrated in Figure 1.

Accordingly, the present invention provides the use of the compound of formula I, or a salt or a solvate thereof, or the combination described herein, for detecting a platinum complex in a test sample.

The present invention also provides a method for detecting a platinum complex in a test sample, the method comprising:
- contacting a test sample suspected of comprising a platinum complex with the compound of formula I, or a salt or a solvate thereof;
- exposing the test sample to a source of light sufficient to excite the compound of formula I; and
- detecting fluorescence from the compound of formula I, wherein the presence of fluorescence indicates the presence of a platinum complex in the test sample.

The present invention also provides a method for detecting a platinum complex in a test sample, the method comprising:
- contacting the test sample with the combination of the compound of formula I, preferably la, and one or more of the compounds of formulae II-VI, VIII, IX, XI-XIII, XVIII and XIX described herein;
- exposing the test sample to a source of light sufficient to excite the compounds of the combination; and
- detecting fluorescence from the compounds of the combination, wherein the presence of fluorescence indicates the presence of a platinum complex in the test sample;
wherein the fluorescence from the compound of formula I and the one or more other fluorogenic sensors compounds together provide a fluorescence response pattern for the platinum complex.

The test sample may be a complex sample, i.e. a sample containing a mixture of two or more analytes which are metal ion complexes. In some embodiments, the test sample comprises the platinum complex and one or more metal complexes other than the platinum metal complex. In some embodiments, the one or more other metal complexes comprises one or more other platinum complexes. Advantageously, as shown in the Examples, the compound of formula I and the combination described herein are capable of discriminating platinum complexes from other metal ion complexes, including other platinum complexes.

The test sample may be a biological sample, an environmental sample or an industrial sample. In some embodiments, the test sample is a biological sample, such as a sample obtained from a human. Examples of suitable biological samples include serum, plasma, saliva, tear fluid, urine and tissue. Advantageously, as shown in the Examples, the compound of formula I and the combination described herein are capable of detecting, identifying and quantifying platinum complexes in complex biological samples such as plasma. In some embodiments, the test sample is an environmental sample, such as a sample obtained from a body of water or earth. The environmental sample may be contaminated with one or more metal ions. Examples of suitable environmental samples include water and soil.

The test sample may be suspected of comprising a platinum complex, that is, it may be known or unknown whether the test sample comprises a platinum complex. Accordingly, the present invention also provides a method for detecting whether a platinum complex is present in a test sample.

As used herein, the term "detecting a platinum complex" includes identifying the platinum complex and quantifying or determining the concentration of the platinum complex in the test sample. The compound of formula I and the combination described herein may be useful for identifying and/or quantifying a platinum complex in a test sample obtained from a source, such as a biological, environmental or industrial source.

Accordingly, the present invention provides the use of the compound of formula I, or a salt or a solvate thereof, or the combination of the compound of formula I and one or more of the compounds of formulae II-IV, VIII, IX, XI-XIII, XVIII and XIX described herein, for identifying a platinum complex in a test sample.

The present invention also provides a method for identifying a platinum complex in a test sample, the method comprising:
- providing a test sample suspected of comprising a platinum complex;
- contacting the test sample with the compound of formula I, or a salt or a solvate thereof;
- exposing the test sample to a source of light sufficient to excite the compound of formula I;
- detecting a fluorescence response from the compound of formula I;
- comparing the fluorescence response of the test sample with a reference data set in the form of a fluorescence response from one or more reference samples each comprising a metal ion complex to identify whether there is a difference in the fluorescence response as between the test sample and the one or more reference samples; and
- determining the identity the platinum complex in the test sample.

The present invention also provides a method for identifying a platinum complex in a test sample, the method comprising:
- providing a test sample suspected of comprising a platinum complex;
- contacting the test sample with the combination of the compound of formula I and one or more of the compounds of formulae II-VI, VIII, IX, XI-XIII, XVIII and XIX described herein;
- exposing the test sample to a source of light sufficient to excite the compounds of the combination;
- detecting a fluorescence response pattern from the compounds of the combination;
- comparing the fluorescence response pattern of the test sample with a reference data set in the form of fluorescence response patterns from one or more reference samples each comprising a metal ion complex to identify whether there is a difference in the fluorescence response pattern as between the test sample and the one or more reference samples; and
- determining the identity of the platinum complex in the test sample.

In some embodiments, the method further comprises providing a reference data set in the form of fluorescence response patterns from one or more reference samples each comprising a metal ion complex. It will be appreciated that the one or more reference samples preferably each comprise a metal ion complex different from that of the other reference samples.

The reference data set contains data on the fluorescence response pattern(s) of one or more reference samples each comprising a metal ion complex. In this context, the chemical structure (identity) of the metal ion complex(es) in the one or more reference samples is known. A reference data set may be obtained by contacting one or more reference samples each containing a metal ion complex with the compound of formula I or the combination described herein, and detecting a fluorescence response pattern for each reference sample from the compound of formula I or the compounds of the combination.

The chemical structure (identity) of the platinum complex in the test sample may be known or unknown. Further, the chemical structure of the platinum complex in the test sample may be the same or different to the metal ion complex(es) of the one or more reference samples. If the fluorescence response pattern of the test sample is the same as a fluorescence response pattern of a metal ion complex in the one or more reference samples, the platinum complex is determined as having a "known" structure that is the structure of the metal ion of that reference sample. If the fluorescence response pattern of the test sample is different to the fluorescence response patterns of the metal ion complex(es) in the one or more reference samples, the platinum complex is determined as having an "unknown" structure that is different to each of the metal ion complexes in the reference samples.

The test sample may comprise one or more metal complexes other than the platinum complex. Accordingly, the present invention also provides a method for discriminating a platinum complex from one or more other metal complexes in a test sample. The one or more other metal complexes may comprise one or more other platinum complexes. Accordingly, the present invention also provides a method for discriminating a platinum complex from one or more other platinum complexes in a test sample.

In embodiments where the test sample comprises one or more metal complexes other than the platinum complex, the one or more other metal complexes each interact the compound of formula I or the compounds of the combination to provide a fluorescence response or fluorescence response pattern that is different to the corresponding fluorescence response or fluorescence response pattern of the platinum complex.

Advantageously, as shown in the Examples, the compound of formula I and the compounds of the combination described herein are capable of discriminating a platinum complex from other metal ion complexes including other platinum complexes, and also exhibit sensitivity to the coordination environment of various metal ions including platinum. It will be appreciated that the compound of formula I and the combination described herein may be useful for detecting, identifying and quantifying any platinum complex in test samples, including complex biological, environmental and industrial samples.

Array-based sensing systems that contain multiple analytes and sensors often generate large multidimensional data-sets. The data may be deconvoluted by using multivariate analysis. Multivariate analysis may be carried out using statistical programs known in the art, for example SPSS, BioVinci, R or Matlab. Advantageously, multivariate statistical techniques may improve data visualisation by reducing dimensionality and allowing identification of trends and predictability in data-sets. Examples of suitable techniques include principal component analysis (PCA) and linear discriminant analysis (LDA). PCA extracts principal components (factors) which best separate the data and helps identify the sensor elements which contribute to the greatest discrimination and still account for most of the variance. LDA is a classification technique that constructs a set of orthogonal, linear combinations of variables which maximise the distances between different classes of data and concentrate this information in a smaller number of dimensions. Once an initial model has been trained, a second set of independent data can be tested in the training matrix to validate the accuracy of the array, and subsequently used to classify unknown data. Other examples of suitable techniques include hierarchical cluster analysis (HCA), artificial neural networks, and linear transformation (regression) techniques such as reduced major axis (RMA) regression.

Accordingly, in preferred embodiments, the methods of the invention further comprise analysing the fluorescence response pattern using multivariate analysis. In some embodiments, the multivariate analysis is selected from principal component analysis (PCA), linear discriminant analysis (LDA), hierarchical cluster analysis (HCA), artificial neural networks and reduced major axis (RMA) regression, especially PCA and LDA.

As shown in the Examples, multivariate analysis techniques may be useful for classifying, for example, different metal ion complexes, including different platinum complexes, and different concentrations of metal ion complexes in one or more test samples. The compound of formula I or the compounds of the combination described herein may be used to provide a training matrix for the multivariate analysis, which is based on a reference data set in the form of fluorescence response patterns from one or more reference samples described herein. Accordingly, the present invention provides the use of the compound of formula I, or a salt or solvate thereof, or the combination of formula I and one or more compounds of formulae II-IV, VIII, IX, XI-XIII, XVIII and XIX described herein, for providing a reference data set for the multivariate analysis. The present invention further provides a method for providing a training matrix for the multivariate analysis comprising providing a reference data set in the form of fluorescence response patterns from one or more reference samples as described herein, and analysing the reference data set using multivariate analysis to provide the training matrix.

The present invention also provides the use of the compound of formula I, or a salt or a solvate thereof, or the combination of the compound of formula I and one or more of the compounds of formulae II-IV, VIII, IX, XI-XIII, XVIII and XIX described herein, for determining the concentration of a platinum complex in a test sample.

The present invention also provides a method for determining the concentration of a platinum complex in a test sample, the method comprising:
- providing a test sample comprising the platinum complex;
- contacting the test sample with the compound of formula I, or a salt or a solvate thereof;
- exposing the test sample to a source of light sufficient to excite the compound of formula I;
- detecting a fluorescence response from the compound of formula I;
- comparing the fluorescence response of the test sample with a reference data set in the form of a fluorescence response from one or more reference samples each comprising a concentration of the platinum complex to identify whether there is a difference in the fluorescence response pattern as between the test sample and the one or more reference samples; and
- determining the concentration of the platinum complex in the test sample, where the fluorescence response of the platinum complex in the test sample is the same as the fluorescence response of the reference sample having the same concentration .

The present invention also provides a method for determining the concentration of a platinum complex in a test sample, the method comprising:
- providing a test sample comprising the platinum complex;
- contacting the test sample with the combination of the compound of formula I and one or more of the compounds of formulae II-IV, VIII, IX, XI-XIII, XVIII and XIX described herein;
- exposing the test sample to a source of light sufficient to excite the compounds of the combination;
- detecting a fluorescence response pattern from the compounds of the combination;
- comparing the fluorescence response pattern of the test sample with a reference data set in the form of fluorescence response patterns from one or more reference samples each comprising a concentration of the platinum complex to identify whether there is a difference in the fluorescence response pattern as between the test sample and the one or more reference samples; and
- determining the concentration of the platinum complex in the test sample, where the fluorescence response pattern of the platinum complex in the test sample is the same as the fluorescence response pattern of the reference sample having the same concentration.

In some embodiments, the method further comprises providing a reference data set in the form of fluorescence response patterns from one or more reference samples each comprising a concentration of the platinum complex. It will be appreciated that the one or more reference samples preferably each comprise a concentration of the platinum complex different from that of the other reference samples.

The reference data set contains data on the fluorescence response pattern(s) of one or more reference samples each comprising a concentration of the platinum complex. In this context, the concentration of the platinum complex in the one or more reference samples is known. A reference data set may be obtained by contacting one or more reference samples each containing a concentration of the platinum complex with the compound of formula I or the combination described herein, and detecting a fluorescence response pattern for each reference sample from the compound of formula I or the compounds of the combination.

The compound of formula I and the combination of the compound of formula I and one or more of the compounds of formulae II-IV, VIII, IX, XI-XIII, XVIII and XIX described herein may be useful for monitoring the concentration of a platinum complex obtained from a source over a period of time.

Accordingly, the present invention provides a method for monitoring the concentration of a platinum complex from a source over a period of time, the method comprising:
- determining the concentration of the platinum complex in a test sample obtained from the source at a time point according to the method for determining the concentration of a platinum complex described herein.

The method may further comprise obtaining the test sample from the source.

The compound of formula I and the combination described herein may be useful for monitoring the concentration of a platinum-containing drug in a subject over the course of a treatment regimen.

Accordingly, the present invention provides a method for monitoring the concentration of a platinum-containing drug in a subject who is receiving a treatment regimen comprising the platinum-containing drug, the method comprising:
- providing a biological sample obtained from the subject during the treatment regimen; and
- determining the concentration of the platinum-containing drug in the biological sample according to the method for determining the concentration of a platinum complex described herein.

The method may further comprise obtaining the biological sample from the subject during the treatment regimen.

The platinum-containing drug may be any suitable platinum-containing drug for treating a disease in the subject. In some embodiments, the platinum-containing drug is an anti-cancer drug. Examples of suitable anti-cancer drugs include cisplatin, oxaliplatin and carboplatin, as well as new candidate drugs.

Advantageously, as shown in the Examples, the compound of formula I and the combination described herein is capable of monitoring the concentration of platinum anti-cancer drugs using complex samples such as plasma obtained from cancer patients during chemotherapy treatment. Determining the concentration of platinum-containing drug may allow for the identification or prediction of the likelihood of toxicity from the drug based on the concentration of the drug in the biological sample. Accordingly, the method may further comprise identifying or predicting the likelihood of toxicity from the platinum-containing drug in the subject, based on the concentration of the drug in the sample.

In addition, the method may further comprise adjusting the dose of the platinum-containing drug being administered to the subject during the treatment regimen, based on the concentration of the drug in the biological sample. For example, if the concentration of the platinum-containing drug is found to be too low for the desired therapeutic response in the subject, the dose of the drug may be suitably increased. On the other hand, if the concentration of the platinum-containing drug is found to be at levels that may lead to toxicity, the dose of the drug may be suitable decreased.

The present invention therefore may advantageously allow for direct monitoring of platinum drugs such as anti-cancer drugs in a subject during a treatment regimen, whereby doses of the drug could be adjusted to achieve a desired concentration range of the drug accordingly (rather than, for example, due to dose-limiting toxicity).

The present invention also provides a kit comprising the compound of formula I, especially the compound of formula IA, or a salt or a solvate thereof, or the combination of the compound of formula I, especially the compound of formula IA, and one or more of the compounds of formulae II-IV, VIII, IX, XI-XIII, XVIII and XIX, especially the compounds of formulae II-VI, described herein. The kit may be for detecting a platinum complex in a test sample, identifying a platinum complex in a test sample, determining the concentration of a platinum complex in a test sample, monitoring the concentration of a platinum complex in a test sample, or monitoring the concentration of a platinum-containing drug in a subject who is receiving a treatment regimen comprising the platinum-containing drug, as described herein.

The kit of the present invention may further comprise components used to perform a sensor-based array. In some embodiments, the kit further comprises a microplate for a fluorescence-based array, such as a 96-well microplate or a 384-well microplate. In these embodiments, the compound of formula I or the compounds of the combination described herein may be provided separately to the microplate. Alternatively, the compound of formula I or the compounds of the combination may be included in one or more wells of the microplate. In the case of the combination, the microplates may include each compound of the combination in separate wells. Alternatively, the microplates may include two or more compounds of the combination together in a single well, preferably where the two or more compounds each contain different fluorophores. This is because it may be difficult to independently detect the fluorescence of two or more compounds in a single well which have the same fluorophore (and therefore may exhibit fluorescence at similar wavelengths). For example, in the case of a combination comprising the compounds of formulae I-VI, preferably the compounds of formulae I, III and IV (rhodamine fluorophore) are not included together in a single well and the compounds of formulae V and VI (coumarin fluorophore) are not included together in a single well.

In some embodiments, the kit further comprises one or more metal ion complexes, which may be used to prepare a reference sample. In these embodiments, the one or more metal complexes may be provided separately to be microplate. Alternatively, the one or more metal complexes may be present in one or more separate wells of the microplate.

In some embodiments, the kit further comprises a solvent, which may be used for dissolving the compounds of the kit and/or the metal ion complexes of the reference samples. Examples of suitable solvents include dimethyl sulfoxide (DMSO) and dimethylformamide (DMF).

In some embodiments, the kit further comprises a buffer for performing the array. Examples of suitable buffers include (4-(2-hydroxyethyl)-1-piperazineethanesulfonic acid) (HEPES) and phosphate-buffered saline (PBS).

Kits useful for carrying out the present invention may also further comprise instructions (e.g., printed instructions) for carrying out a method as described herein, optionally packaged together in a common package or container.

It will be understood that the invention disclosed and defined in this specification extends to all alternative combinations of two or more of the individual features mentioned or evident from the text or drawings. All of these different combinations constitute various alternative aspects of the invention.

### Examples

The invention will be further described by way of non-limiting example(s). It will be understood to persons skilled in the art of the invention that many modifications may be made without departing from the scope of the invention.

The practice of the present invention employs, unless otherwise indicated, conventional organic synthesis, analysis and molecular characterisation techniques within the skill of the art. Such techniques are well known to the skilled worker, and are explained fully in the literature.

Chemicals for the synthesis of fluorescent sensors and platinum complexes were purchased from Sigma Aldrich and Alfa Aesar. HEPES buffer and PBS buffer powders were purchased from Sigma Aldrich and made using Milli-Q water. Products were purified by flash column chromatography using Davisil 230-400 mesh Kieselgel 60 silica eluting with solvent as described. Solvents were purchased from commercial sources and were purified through a Pure Solv 499-5MD solvent purification system (Innovative Technology, Inc).

Proton Nuclear Magnetic Resonance Spectroscopy (¹H NMR) Spectra were recorded at 300 K using Bruker Avance 300 at a frequency of 300 mHz. Carbon Nuclear Magnetic Resonance Spectroscopy (¹³C NMR) Spectra were recorded on Bruker Avance 400 at a frequency of 75 MHz. Low resolution electrospray ionisation (ESI) mass spectrometry (MS) was performed on a Bruker AmaZon SL ion trap mass spectrometer. High resolution mass spectrometry was performed on a Bruker Apex qE 7T Fourier Transform Ion Cyclotron Resonance mass spectrometer.

### Example 1. Synthesis of the compound of formula IA.

The compound of formula IA (herein S3) was synthesised using the following procedure. To a solution of methyl carbamimidothioate hemisulfate (500 mg, 3.6 mmol) in 2 M NaOH (3 mL) was added toluene (5 mL) and di-tert-butyl dicarbonate (0.39 g, 2.20 mmol). The reaction mixture was heated to 60 °C and stirred for 16 h. The organic layer was separated, washed with brine (20 mL), dried (Na₂SO₄) and the solvent evaporated *in vacuo* to afford the product (0.33 g, 1.7 mmol) as an off-white solid. ¹H NMR (300 MHz, CDCl₃): δ 7.51 (br s, 1 H), 2.46 (s, 3 H), 1.51 (s, 9 H). ¹³C NMR (75 MHz, CDCl₃): δ 173.0, 161.7, 79.9, 28.2, 13.5. LRMS (ESI): calculated for [M+H]⁺ (C₇H₁₄N₂O₂S) m/z: 191.0 found [M+H]⁺: 191.0, found [M-*t*-Bu+H]⁺: 135.0.

A solution of Rhodamine B (100mg, 0.21 mmol) and thionyl chloride (0.30 mL, 4.11 mmol) was stirred at room temperature for 2 h. The acid chloride was precipitated in diethyl ether, filtered and washed with ether. The viscous oil was dissolved in 10 mL dry MeCN and added dropwise to a solution of boc-protected 2-methyl-2-thiopseudourea (45 mg, 0.24 mmol), K₂CO₃ (150 mg, 1.09 mmol) in MeCN (3 mL). The reaction mixture was stirred at room temperature for 16 h. The product was filtered through a silica plug (50:50 EtOAc:hexane) and the solvent evaporated *in vacuo* to afford a violet oil. The product was redissolved in CH₂Cl₂ (6 mL) and trifluoroacetic acid (1 mL) was added. The reaction was stirred at room temperature for 1 h, the solvent removed *in vacuo* and the product was purified using flash chromatography (SiO₂, 2:98 MeOH:CH₂Cl₂) to yield a light pink solid (48 mg, 0.093 mmol). ¹H NMR (500 MHz; CDCl₃): δ 7.93 (d, J = 7.5 Hz, 1H), 7.47 (td, J = 7.4 Hz, 1.1 Hz, 1H), 7.42 (td, J = 7.5 Hz, 1.0 Hz, 1H), 7.01 (d, J = 7.5 Hz, 1H), 6.52 (d, J = 8.8 Hz, 2H), 6.39 (d, J = 2.6 Hz, 2H), 6.26 (dd, J = 8.9 Hz, 2.6 Hz, 2H), 3.33 (q, J = 7.2 Hz, 8H), 2.13 (s, 3H), 1.16 (t, J = 7.3 Hz, 12H). ¹³C NMR (125 MHz, CDCl₃): δ 12.8, 14.3, 44.4, 67.0, 97.9, 106.6, 107.9, 123.8, 124.3, 127.9, 128.1, 128.4, 128.5, 134.5, 149.0, 152.9, 154.2, 169.2; HRMS (ESI): calculated for [M+H]⁺ (C₃₀H₃₃N₄O₂S) m/z: 514.2402 found [M+H]⁺: 514.2477.

### Example 2. Synthesis of other fluorogenic sensors.

The compound of formula II (herein S1) was synthesised from fluorescein in two steps, first by amidating in to give fluorescein hydrazide, and then heating in the presence of CS₂ and KOH. Subsequent acidification gave the final product.

The compound of formula X was synthesised using the following procedure. Indazole (0.69 g, 5.80 mmol) was stirred in ethanol (10 mL) in the presence of potassium hydroxide (0.34 g, 5.80 mmol) for 10 min, after which carbon disulfide (0.45 mL, 7.54 mmol) was added dropwise, with rapid formation of a bright orange precipitate. The precipitate was separated by filtration and resuspended in water. The suspension was acidified to pH 4 with 2M HCl, yielding a precipitate which was subsequently filtered and washed with cold ethanol, to give 1H-indazole-1-carbodithioic acid as a pale orange powder (0.87 g, 78%); mp 220 °C (No reported value). δ H (300 MHz; DMSO-d₆) 9.15 (d, 1 H, J 8.5), 8.31 (s, 1 H), 7.89 (d, 1 H, J 8.5), 7.64 (t, 1 H, J 14), 7.41 (t, 1 H, J 14) ppm. δ C (300 MHz; DMSO-d₆) 218.0, 139.1, 134.9, 127.1, 126.8, 122.2, 120.9, 117.9 ppm. υₘₐₓ(neat): 1738, 1610, 1268, 1008, 908, 849 cm⁻¹. λₘₐₓ(log10ε) (DMSO) 360 (3.88), 325 (3.98). *m*/*z* (LREI): calculated for [M+H]+: 194.00, found: 193.78.

The compound of formula XIV was synthesised using the following procedure. Benzylamine (1.35 g, 12.6 mmol) was added to a solution of 2-amino-1-cyclopentene-1-carbodithioic acid (ACDA, 2.0 g, 12.6 mmol) in methanol (40 mL). The reaction mixture was stirred at room temperature for 48 h, after which water was added to the reaction flask and the mixture immediately filtered. The filtrate was cooled to 0 °C and neutralised with 2M HCl to give a crystalline yellow precipitate which was subsequently filtered and washed with cold methanol. The product was recrystallised from methanol to yield 2-(benzylamino)cyclopent-1-enecarbodithioic acid as dry yellow needles (1.8 g, 57%); mp 100 °C (Lit. 98-99 °C [98]). δ H (300 MHz; DMSO-d₆) 12.44 (br. s, 1 H), 7.36-7.16 (m, 5 H), 4.61 (d, 2 H, J 6.0), 4.05 (br. s, 1 H), 2.76 (t, 2 H, J 7.5), 2.67 (t, 2 H, J 7.0), 1.71 (quin, 2 H, J 7.5) ppm. δ C (75 MHz; DMSO-d₆) 192.2, 168.1, 137.6, 128.8, 128.7, 128.4, 127.4,127.3, 24.3, 34.9, 33.0 ppm. υₘₐₓ(neat): 3325 (br), 3029, 2545, 1584, 1485, 1375, 1326, 1275, 1007, 921, 875 cm-1. λₘₐₓ(log10ε)(DMSO) 400 (4.45), 305 (3.97). *m*/*z* (LREI): calculated for [M+H]⁺: 250.07, found: 249.90.

The compound of formula XX was synthesised using the following procedure. Intermediate compound 3,6,12,15-tetrathia-9-monoazaheptadecane was synthesised using the procedure described in Zeng et. al. (Journal of the American Chemical Society 128 (2006): 10-11) and was used in a subsequent reaction without further purification. 3,6,12,15-Tetrathia-9-monoazaheptadecane (660 mg, 2.1 mmol) was stirred in ethanol (30 mL) in the presence of formaldehyde (excess, 1 mL) at 60 °C for 1 h. 4-Methylumbilliferone (350 mg, 2.0 mmol) was then added and the reaction mixture was heated to reflux and allowed to stir for 12 h. The reaction mixture was evaporated in *vacuo* then taken up in chloroform (200 mL) and washed sequentially with water (100 mL) and brine (75 mL), dried (Na₂SO₄) and evaporated *in vacuo.* The product was purified by flash chromatography on silica, eluting with methanol-dichloromethane (1:9) to give 8-((bis(2-((2-(ethylthio)ethyl)thio)ethyl)amino)methyl)-7-hydroxy-4-methyl-2H-chromen-2-one as a light orange oil (590 mg, 59%). δ H (400 MHz; DMSO-d₆) 7.58 (d, 1 H, J 9.0), 6.78 (d, 1 H, J 9.0), 54 6.11 (s, 1 H), 4.68 (s, 2 H), 3.68-3.65 (m, 4 H), 3.55-3.42 (m, 12 H), 2.69-2.58 (m, 4 H), 2.36 (s, 3 H), 1.17 (t, 6 H, J 7.5) ppm. δ C (100 MHz; DMSO-d₆) 160.7, 160.3, 154.2, 154.0, 126.4, 125.7, 113.1, 112.7, 112.4, 65.5, 60.0, 55.3, 32.0-31.1, 19.0, 15.6 ppm. υₘₐₓ(neat): 3215 (br), 2972, 2975, 1719, 1600, 1578, 1384, 1367, 1065, 928, 845 cm⁻¹. λₘₐₓ(log10 ε)(DMSO) 340 (4.23). *m*/*z* (LRAPCI): calculated for [M+H]⁺: 502.16, found: 502.36.

The compound of formula III (herein S2) was synthesised using the procedure described in Kolanowski et. al. (Sensors and Actuators B, 2018, 255, 2721-2724). The compound of formula IV (herein S4) was synthesised using the procedure described in Shi et. al. (Chem. Commun., 2008, 1856-1858). The compound of formula VI (herein S6) was synthesised using the procedure described in Kuar et. al. (Chem. Commun., 2015, 51, 10510-10513). The compound of formula VII was synthesised using the procedure described in Smith et. al. (Analyst 2019, 144 (1), 230-236). The compound of formula VIII was synthesised using the procedure described in Kim et. al. (Org. Lett. 2010, 12 (22), 5342-5345), except using Rhodamine B instead of Rhodamine 6G. The compound of formula IX was synthesised using the procedure described in Chen et. al. (Tetrahedron 2010, 66, 4016-4021). The compound of formula XI was synthesised using the procedure described in Kai et. al. (Sensors Actuators B Chem. 2014, 202, 252-256). The compound of formula XVI was synthesised using the procedure described in Macas et. al. (Polyhedron 21 (2002): 1229-1234). The compound of formula XVII was synthesised using the procedure described in Safin (Dalton Trans., 2013,42, 4757-4763). The compound of formula XVIII was synthesised using the procedure described in Lee et. al. (Organic Letters 10 (2008): 213-216). The compound of formula XIX was synthesised using the procedure described in Bag and Bharadwaj (The Journal of Physical Chemistry B 109 (2005): 4377-4390).

The compound of formula V (herein S5), the compound of formula XII (herein S8), the compound of formula XIII (herein S9) and the compound of formula XV were purchased from Sigma Aldrich.

### Example 3. Synthesis of platinum compounds

Platinum complexes cisplatin, transplatin, [PtCl₂(en)], phenanthriplatin, [PtCl(NH₃)₃]Cl, K₂[PtCl₄], oxaliplatin and pyriplatin were synthesised via previously-reported methods: Dhara, A Rapid Method for the Synthesis of Cis[Pt(NH3)2Cl2], Indian J. Chem. 1970, 8, 193-194; Kauffman, Cis- and Trans-Dichloroammine-Platinum (II), 1963, VII, 239-245; Chopade et. al., Synthesis, Characterization, Structures and Cytotoxicity of Platinum(II) Complexes Containing Dimethylpyrazole Based Selenium Ligands, Inorganica Chim. Acta 2015, 427, 72-80; Park, G et. al., Phenanthriplatin, a Monofunctional DNA-Binding Platinum Anticancer Drug Candidate with Unusual Potency and Cellular Activity Profile, Proc. Natl. Acad. Sci. U. S. A. 2012, 109 (30), 11987-11992; and Morita et. al. Triamminechloroplatinum(II) Chloride. Inorg. Synth. 1983, 22, 124-125.

### Example 4. Fluorescence array procedure

Fluorescence spectroscopy was performed on a bench top Perkin-Elmer Enspire Multimode Plate Reader. Arrays were prepared by mixing stock solutions of the metal salts or metal complexes with sensors S1-S9 or S1-S6 and DMF in flat-bottomed black polypropylene 96-well microplates to create a multidimensional array. 5 replicates of each sensor-metal or sensor-complex solution were used. Four excitation wavelengths were selected based on the maximum excitation wavelengths for the sensors S1-S9: 280 nm, 380 nm, 480 nm, 445 nm and 545 nm, with emission intensity ranges 300-500nm, 400-600nm, 500-650nm, 465-600nm, 565-700nm respectively. Fluorescence responses to analytes were determined using integrated emission intensity, and for each sensor a control well containing no analyte was included to normalise the response for analysis.

To facilitate interpretation of the multidimensional data, dimensionality reductions were used to deconvolute data. Principal Component Analysis (PCA) and Linear Discriminant Analysis (LDA) were performed using IBM SPSS Statistics 25. Normalised integrated emission response to each analyte was subjected to multivariate analysis, with analytes input as the grouping variable and fluorescence responses of each sensor as independent variables. Dimension reduction was performed using PCA with no selection variable, during which the number of principal components extracted was based on a scree plot, where values underwent a varimax rotation and eigenvalues >1 were retained. Classification was determined using LDA, using analyte class as the grouping variable and all groups classified as equal. Data was interpreted by territorial maps and canonical score plots of the first 2 discriminant functions. A confusion matrix was used to determine classification accuracy and validated using a leave-one-out or jackknife cross-validation routine.

### Example 5. Identification of sensors for fluorescence array.

To identify sensors useful for detecting platinum complexes in test samples, arrays were conducted using sensors selected from the compounds of formulae I-XX. The sensors were tested using the procedure described in Example 4, and the obtained data was analysed using LDA. The combination of the compounds of formulae I-VI, VIII, XII and XIII (sensors S1-S9), particularly the combination of the compounds of formulae I-VI (sensors S1-S6), were identified to contribute to the best discrimination while still accounting for most of the variance. S1-S9 were found to have intense fluorescence in the visible range between 400-600 nm: S1 excited at 480 nm, S2-4 excited at 545 nm, S5 excited at 380 nm, S6-S7 excited at 445 nm and S8-S9 excited at 280 nm. Sensors S1-S9 were used in subsequent experiments.

### Example 6. Discrimination of a platinum complex from other metal complexes

To assess whether the sensor array was capable of discriminating a platinum complex in a sample containing a variety of metal ions, including some heavy metals, sensors S1-S9 were screened against various metal ions, including heavy metal ions, using the procedure described in Example 4. Each probe in the array (10 µM) was screened with nine metal ions (50 µM), namely Ni(II), Cu(II), Zn(II), Fe(III), Co(II), Pb(II), Cd(II), Hg(ll) and Pt(II), in 20 mM HEPES, pH 7.4. The fluorescence spectra of the array was recorded. The response of the six sensors with each metal ion produced a distinct pattern, and this pattern was clarified using multivariate analysis.

Figure 2a illustrates the fingerprint response of each sensor to the nine metal ions, including both quenching and enhancement of fluorescence. The results show good cross-reactivity and fluorescence response of the sensors towards metal ions.

The responses from sensors S1-S9 and metal ions were subjected to PCA. Some clustering of sample classes was observed, indicating a supervised technique such as LDA may have good discrimination accuracy.

Figure 2b illustrates the LDA score plot of the first three component scores for sensors S1-S9. The LDA plot captured more variance in the reduced dimensions compared to PCA, with the first three factors extracted representing 99% of the variance. In addition, the LDA model was able to classify all metal replicates correctly with 98% accuracy and 93% accuracy using a leave-one-out cross validation routine. Factor 1 represented almost all the variance (91.7%), which may be due to the skewed loading of Hg(ll) ions.

It was explored whether the number of sensors in the array could be reduced, to improve the sensor-to-analyte ratio. The LDA results were used to determine which sensors were contributing the least toward the discrimination. To do this, the loading of each sensor onto the first three LDA factors, as the first three factors represented 99.5% of the variance. A greater loading on the higher factors may indicate that the sensor is contributing more toward discrimination.

The loading scores for the nine sensor system were extracted and sensors with strong factor loadings identified. S1, S2 and S4 had a high coefficient for Factor 1, S3 and S5 had a high coefficient for Factor 2, and S6 and S9 had a high coefficient for Factor 3. In contrast, neither S7 nor S8 had high coefficients for the first 3 factors. Whilst S9 loaded well onto Factor 3, this factor represented only 0.5% of the variance and the sensor demonstrated virtually no response towards platinum ions. Thus, the sensor array system was reduced to six sensors, S1-S6, and was then investigated for its metal ion discrimination ability.

The first three component scores from the PCA plots for sensors S1-S6 showed that 83.2% of the variance could be explained by these factors alone. Initial descriptive statistics suggested that S2 and S4 may be strongly correlated. Removal of either sensor resulted in poorer sampling adequacy, therefore both were retained for subsequent discriminant analysis.

The LDA plot showed better clustering of data, and analysis could distinguish all nine metal ions with 93% classification accuracy (91% cross-validated). Importantly, the array was able to correctly classify and discriminate all of the Pt (II) replicates from the eight other metal ions. This was noteworthy, as there are array-based sensing systems that discriminate biological metals and heavy metals but these rarely include Pt(II).

The LDA plot in Figure 2b shows Hg(ll) clustered away from the rest of the metal ions. This was not surprising, as S4 is a reported Hg(ll) sensor and its unique response to mercury appears to skew the first discriminate score. Further interrogation of the LDA data illustrated that both S2 and S4 have the highest loading the Factor 1. Removing either sensor did not significantly affect accuracy, the cross-validated classification accuracy remaining at 91% with Hg(II) still clustered away from the other metals. Removal of both sensors reduced the classification accuracy to 87%. These results suggest that that selective sensors in array-based sensing may influence discriminant scores.

The results of this experiment indicate that the sensor array comprising S1-S9, particularly S1-S6, may be useful for distinguishing platinum from other biologically relevant metals such as nickel, copper, zinc and iron in a complex sample.

### Example 7. Discrimination of various platinum complexes in a test solution.

To assess whether the sensor array was capable of identifying specific platinum complexes in a test solution, a test solution containing various platinum complexes was assessed with the S1-S6 sensor array using the procedure described in Example 4. Each sensor in the array (200 µM) was tested with seven platinum complexes (200 µM), namely cisplatin, transplatin, [PtCl₂(en)], phenanthriplatin, [PtCl(NH₃)₃]Cl, K₂[PtCl₄] and oxaliplatin, in 20 mM HEPES, pH 7.4. The fluorescence spectra of the array was recorded. The response of the six sensors with each platinum complex produced a distinct pattern (Figure 3), and this pattern was clarified using multivariate analysis.

The PCA plots of the first three component scores showed some separation of classes, indicating desirable variability within the data. Importantly, a correlation matrix of the six sensors showed that all probes were not all strongly correlated, and all sensors contributed to the distinguishing power of the array. This suggests that including all 6 sensors in the array may be beneficial.

Figure 4 illustrates the LDA plot of the first three factors obtained. The LDA plot of the first two canonical scores revealed some overlapping of classes, which were better separated by extracting a third factor. Jack-knifed classification with cross-validation revealed that 100% of cases were correctly classified.

The results of this experiment demonstrate that seven platinum complexes of different structure in a test solution were able to be distinguished using the sensor array. The results indicate that the sensor array may be useful for detecting, discriminating and identifying various platinum complexes in a complex sample.

### Example 8. Identification of an unknown platinum complex.

To assess the capability of the S1-S6 sensor array to classify an unknown platinum complex, the data acquired on the "known" platinum complexes tested in Example 7 were used as a training set to examine an "unknown" platinum complex, namely pyriplatin. Pyriplatin is most similar to "known" platinum complex phenanthriplatin, which is also a monofunctional complex with a heterocyclic ligand. The assay was conducted using the procedure described in Example 7.

Figure 5a illustrates the LDA plot of the first three factors when ungrouped cases were processed in LDA, and Figure 5b illustrates the LDA plot when the data was reprocessed specifying that there were eight platinum complexes. When ungrouped cases were processed in LDA, all replicates of pyriplatin were classified as phenanthriplatin. However, when the data was reprocessed, the results correctly classified pyriplatin as an eighth class.

To understand the types of reaction taking place, the average of five replicate responses between each sensor and platinum complex was processed in HCA. Figure 5c is a dendrogram presenting the HCA results. The clusters were paired based on similarities. The first cluster is between pyriplatin and phenanthriplatin, consistent with the previous training array experiments. Secondary clusters separate cis-configurations from non-cis-configurations and these clusters are separate from the heterocyclic monofunctional complexes. Thus, this analysis may suggest the array is sensitive to coordination sphere, and these interactions may contribute to the discriminatory power of the array.

The results of this experiment demonstrate that platinum complexes of different structure in a test solution were able to be distinguished using the sensor array. The results also demonstrate the sensitivity of the array to the coordination environment of the complexes and ability to distinguish between similar complexes. The results indicate that the sensor array may be useful for detecting, discriminating and identifying various platinum complexes in a complex sample. The sensitivity of the array to discriminate between platinum complexes also suggests that the array may be useful for monitoring the presence, metabolism and fate of platinum complexes over time.

### Example 9. Determining platinum drug concentration in spiked plasma samples

To assess whether the S1-S6 sensor array could be used to detect and quantify the presence of a platinum complex in a complex biological sample, the sensor array was tested using human plasma samples spiked with a platinum-containing drug, namely cisplatin or oxaliplatin, using the procedure described in Example 4. The fluorescence spectra of the array was recorded. The response of the six sensors with each platinum drug produced a distinct pattern, which was clarified using multivariate analysis. The platinum concentrations in the blood plasma samples were confirmed by ICP-MS.

First, the platinum drugs were each tested at concentrations between 0.5 µM and 5 µM, which is similar to the concentration range administered to cancer patients during chemotherapy treatment. LDA performed on the responses of the six sensors with each platinum drug at four concentrations showed 79% correct classification (32% cross-validated) for cisplatin and 80% correct classification (30% cross-validated) for oxaliplatin.

To assess whether the inclusion of additional fluorescent measurements could improve classification accuracy, the sensor array was conducted testing the six sensors at 4 excitation wavelengths. The four excitation wavelengths were selected based on the maximum excitation wavelengths for the six sensors: 380nm, 445nm, 480nm and 545nm, with emission intensity ranges 400-550nm, 465-600nm, 500-650nm, 565-700nm, respectively. This provided 24 fluorescent measurements available for multivariate analysis. Optimisation studies, removing data with zero emission intensity and PCA were performed on the additional measurements to identify which extra measurements could be used to improve discrimination. 21 measurements gave improved classification accuracy (100% correct classification) for both cisplatin and oxaliplatin. By closely examining the PCA output, variables with high correlation, similar loadings and low communalities were progressively removed whilst retaining 100% correct classification. The classification accuracy was found to drop below 100% when less than 11 measurement variables were used. Cross-validated accuracy was found to peak at 13 and 14 variables for cisplatin and oxaliplatin, respectively.

For consistency, 14 optimised instrument variables were selected for the subsequent experiments. Figures 6a and 6b illustrate the LDA plots of the first three factors obtained using the optimised system with cisplatin and oxaliplatin, respectively. The results show good discrimination of all concentrations of cisplatin and oxaliplatin tested with 100% classification accuracy. These results indicate that the sensor array may be useful for determining the concentration of platinum complexes in complex biological samples.

The results were further evaluated using multivariate analysis according to both drug type and concentration. For this analysis, cisplatin and oxaliplatin were grouped separately and into two concentration ranges for each drug, 0.5-1 µM and 2-5 µM. The responses were classified using LDA and revealed 90% correct classification (74% cross-validated accuracy). As shown in Figure 6c, the three-dimensional LDA score plot also illustrated non-overlapping clusters of cisplatin and oxaliplatin.

These results demonstrate the ability of the sensor array to discriminate different platinum drugs, including sensitivity to concentration ranges. This is important for clinical applications, where clinical decisions may be made based on concentration ranges, rather than specific concentrations. Thus, a technique able to distinguish between high, medium and low concentrations of platinum chemotherapy drugs in human plasma may be beneficial for clinical and therapeutic drug monitoring applications.

### Example 10. Determining platinum drug concentration in clinical plasma samples.

To assess whether the sensor array could be used to distinguish biological samples based on Pt(II) concentration, the sensor array was tested on plasma samples taken from cancer patients treated with a platinum-containing drug at various stages of chemotherapy treatment.

72 plasma blood samples were collected, 61 from patients treated with oxaliplatin and 11 from patients treated with cisplatin. Patient blood samples were collected in conjunction with routine blood tests, prior to receiving a prescribed cycle of platinum-based cancer treatment. Samples were centrifuged at room temperature for 10 mins at 3500 rpm, and subsequently aliquoted in EDTA microtubes for storage at -80°C.

To determine total platinum concentration in blood plasma samples, samples were defrosted and 50 µL of plasma was digested in 100 µL of 37% analytical grade hydrochloric acid for 3 days at room temperature. Samples were vortexed, centrifuged, then diluted in ultra-pure water using a Hamilton autodiluter to a 100 ppt to 1 ppm concentration range. ICP-MS measurements were performed on a PerkinElmer Nexion 300X mass spectrometer operating in standard mode with 10 s integration time for all elements measured and using a 10ppb Rh/Ir internal standard solution.

The oxaliplatin samples were found to range in concentration from 0 to 504 ppb and the cisplatin samples ranged in concentration from 30 to 1450 ppb. During array preparation, the samples were diluted by a factor of 2. Therefore, the concentration was expected to range from 0 to 252 ppb for oxaliplatin and 15 to 1725 ppb for cisplatin. The diverse range of concentrations allowed for assessment of the sensitivity of the array towards both platinum drug and concentration.

Figure 7a illustrates the output of the array and the classification of the data corresponding to either cisplatin or oxalplatin. The data was correctly classified with 100% accuracy, and 30% LOO (leave-one-out) cross-validated. The low LOO cross-validation score may be due to the diverse concentrations within each grouping. While the cross validation score is less accurate, it was not entirely surprising that the LDA algorithm this was not able to distinguish all the data considering some of the data only falls wither side of the concentration boundaries. To demonstrate this further, a 30 sample subset of the clinical data with no close overlap was selected, with 10 samples in each class and a gap of 20 ppb between each class. The array was then able to classify the data with 93% correct classification and 47% LOO cross-validation. Figure 7b illustrates the two-dimensional LDA score plot of the first two factors. Using 25 samples in the same concentration ranges that achieved 96% correct classification, and using this sub-set as a training set, 11 unknown samples in these ranges were classified with 64% accuracy.

The results show that the sensor array is sensitive to concentration and may be capable of discriminating concentrations of platinum complexes less than 100 ppb. The results indicate that the sensor array may be useful for discriminating between concentrations of platinum complexes in complex samples.

Finally, to assess whether the sensor array was capable of distinguishing samples based on their time of collection, the sensor array was tested with the clinical oxaliplatin samples. Of the 61 oxaliplatin samples, 51 were from the same 17 patients, collected at baseline, interim and final time points. The array measurements have been shown to be sensitive to coordination environment and may therefore be sensitive to the different states of platinum drug that are likely to exist after a period of time in the blood.

The results of the array on samples classified per time point are shown in Figure 8a. The array was able to classify 70% of the samples correctly based on the timepoint of collection with 45.1% LOO cross-validation. The results indicate that the sensor array may be useful for discriminating between samples obtained at different time points.

The clinical samples were also evaluated based on treatment cycle. Samples after a patient's first cycle of treatment, after six cycles of treatment, and 2-4 weeks post the completion of treatment, were analysed by LDA and grouped according to collection time. The LDA score plots are illustrated in Figures 8b and 8c. LDA classified the 39 samples with 80% accuracy (46% cross-validated accuracy) according to the collection time, which suggests there may be similarities in the state of platinum in the bloodstream depending on the stage of treatment. The ICP-MS results of clinical samples showed no obvious correlation between the treatment stage and Pt(ll) concentration, whereas the sensor array was able to discriminate samples based on these time points. This suggests there may be other factors linking the samples at the same stage, such as the ratio of bound and unbound platinum drug over time, highlighting the advantages of using an analytical technique that is sensitive to the coordination environment.

## Claims

1. A compound of formula I: 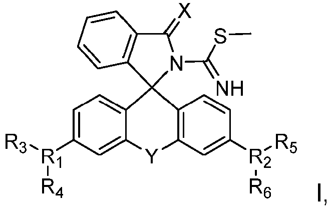 wherein:
X is O or S;
Y is selected from O, Si, Se and Ge;
R₁ and R₂ are independently N or O; and
R₃-R₆ are independently absent or H or C₁-C₆alkyl,
or a salt or solvate thereof.

2. The compound according to claim 1where the compound has the formula Ia: 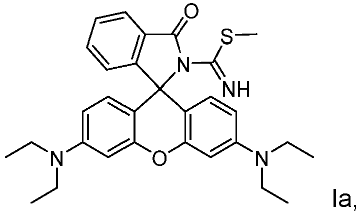 or a salt or solvate thereof.

3. A combination comprising the compound, or a salt or solvate thereof, of claim 1 or 2 and one or more additional fluorogenic compounds.

4. The combination according to claim 3 wherein the one or more additional fluorogenic compounds are selected from:
a compound of formula II: 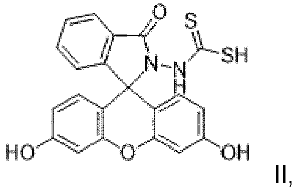
a compound of formula III: 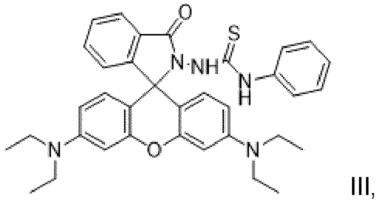
a compound of formula IV: 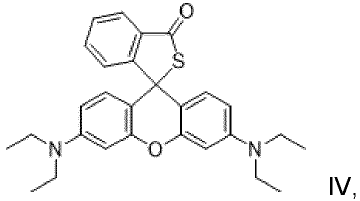
a compound of formula V 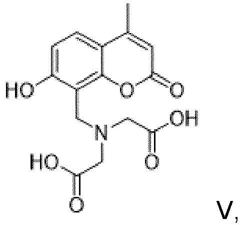
a compound of formula VI: 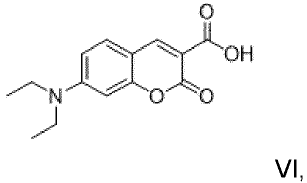
a compound of formula VIII: 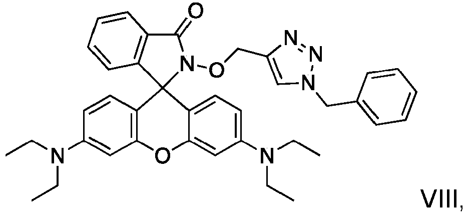
a compound of formula IX: 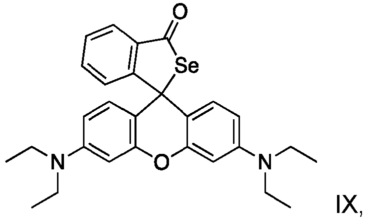
a compound of formula XI: 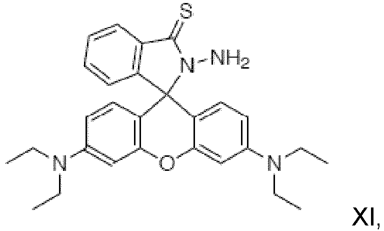
a compound of formula XII: 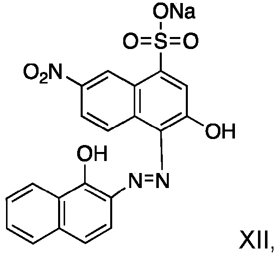
a compound of formula XIII: 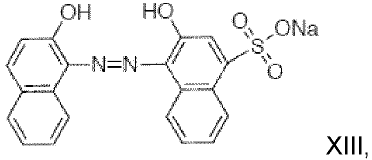
a compound of formula XVIII: 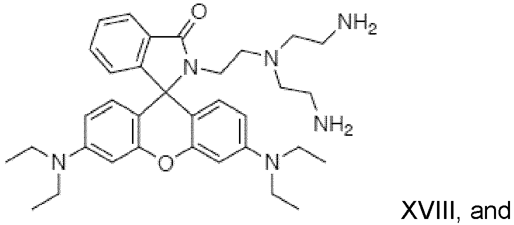
a compound of formula XIX: 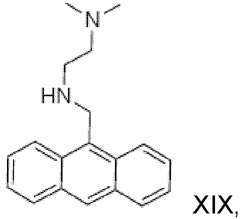
or salts or solvates thereof.

5. The combination of claim 4 wherein the combination further comprises one or more compounds selected from: 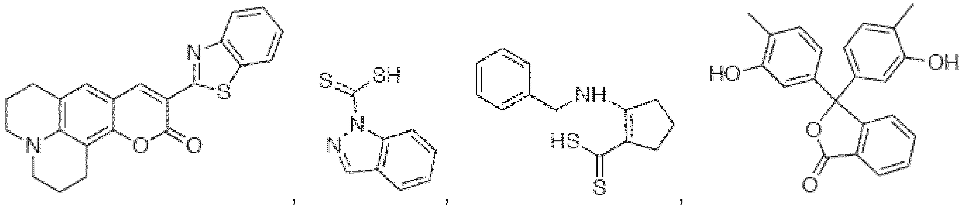 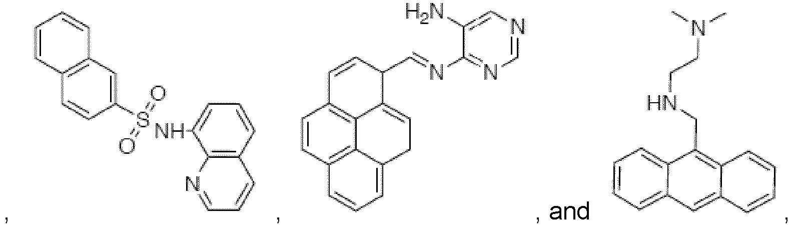 or salts or solvates thereof.

6. The combination of claim 4 or claim 5 wherein the combination comprises:
- the compound of formula I, preferably la, and
- one or more compound(s) selected from the compounds of formulae II-VI, VIII, XII and XIII and salts and solvates thereof, or alternatively one or more compounds selected from the compounds of formulae II-VI, IX, XI, XVIII and XIX, and salts and solvates thereof.

7. The combination of any one of claims 3 to 6, wherein the combination comprises the compound of formula I, preferably la, and one or more compounds of formulae II-VI, or salts or solvates thereof.

8. Use of the compound of claim 1 or claim 2 or the combination of any one of claims 3 to 7, for detecting a platinum complex or combination of platinum complexes in a test sample.

9. A method for detecting a platinum complex in a test sample, the method comprising:
- contacting a test sample suspected of comprising a platinum complex with the compound of claim 1 or claim 2 or the combination of any one of claims 3 to 7;
- exposing the test sample to a source of light sufficient to excite the compound of formula I or the compounds of the combination; and
- detecting fluorescence from the compound of formula I or the compounds of the combination, wherein the presence of fluorescence indicates the presence of the platinum complex in the test sample.

10. A method for identifying a platinum complex in a test sample according to claim 9, where additionally the detection method for compounds of the combination in any one of claims 3 to 7 comprising:
- detecting a fluorescence response pattern from the compounds of the combination;
- comparing the fluorescence response pattern of the test sample with a reference data set in the form of fluorescence response patterns from one or more reference samples each comprising a metal ion complex to identify whether there is a difference in the fluorescence response pattern as between the test sample and the one or more reference samples; and
- determining the identity of the platinum complex in the test sample, preferably where
- the fluorescence response pattern of the platinum complex in the test sample is the same as the fluorescence response pattern of the reference sample having the same concentration.

11. The method of claim 10 wherein the method further comprises analysing the fluorescence response pattern using multivariate analysis.

12. The use of claim 8 or the method of any one of claims 9 to 11 wherein the test sample comprises one or more metal complexes other than the platinum complex, preferably wherein the one or more other metal complexes comprises one or more other platinum complexes.

13. The use or the method of any one of claims 8 to 12 wherein the test sample is a biological sample, preferably wherein the biological sample is selected from serum, plasma, saliva, tear fluid, urine and tissue.

14. A method for monitoring the concentration of a platinum-containing drug, preferably an anti-cancer drug, in a subject who is receiving a treatment regimen comprising the platinum-containing drug, the method comprising:
- providing a biological sample obtained from the subject during the treatment regimen; and
- determining the concentration of the platinum-containing drug in the biological sample according to the method of any one of claims 9 to 13.

15. A kit comprising the compound of claim 1 or claim 2 or the combination of any one of claims 3 to 7.

## Patentansprüche

1. Verbindung der Formel I: 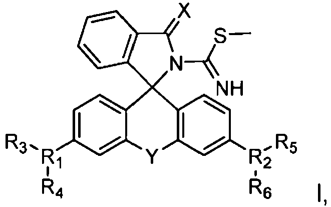 worin:
X O oder S ist,
Y aus O, Si, Se und Ge ausgewählt ist,
R₁ und R₂ jeweils unabhängig voneinander N oder O sind und
R₃-R₆ jeweils unabhängig voneinander fehlen oder H oder C₁-C₆-Alkyl sind;
oder ein Salz oder Solvat davon.

2. Verbindung nach Anspruch 1, wobei die Verbindung die Formel Ia aufweist: 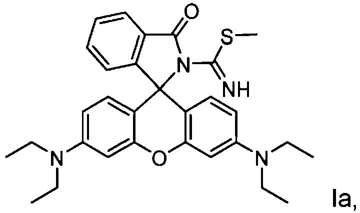 oder ein Salz oder Solvat davon.

3. Kombination, welche eine Verbindung oder ein Salz oder Solvat davon nach Anspruch 1 oder 2 und eine oder mehrere zusätzliche fluorogene Verbindungen umfasst.

4. Kombination nach Anspruch 3, wobei die eine oder mehreren zusätzlichen fluorogenen Verbindungen aus den folgenden ausgewählt sind:
einer Verbindung der Formel II: 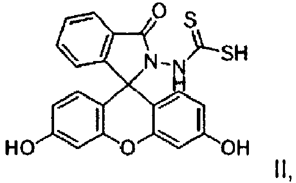
einer Verbindung der Formel III: 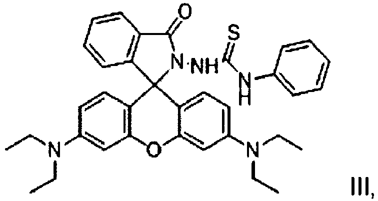
einer Verbindung der Formel IV: 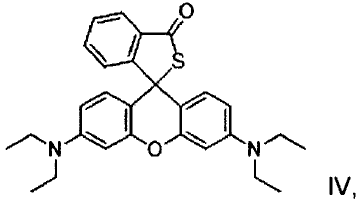
einer Verbindung der Formel V: 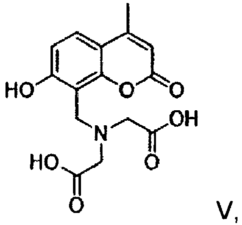
einer Verbindung der Formel VI: 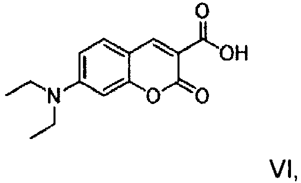
einer Verbindung der Formel VIII: 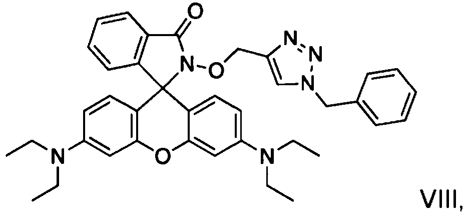
einer Verbindung der Formel IX: 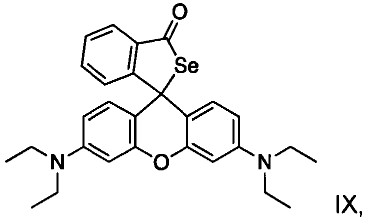
einer Verbindung der Formel XI: 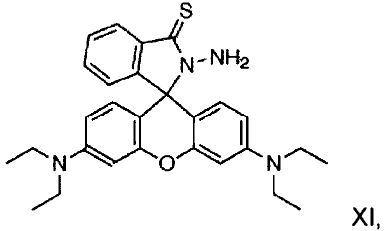
einer Verbindung der Formel XII: 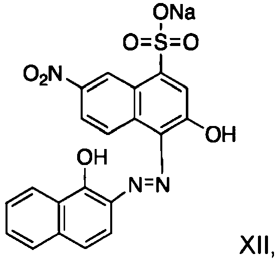
einer Verbindung der Formel XIII: 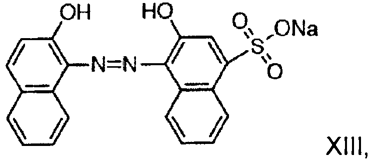
einer Verbindung der Formel XVIII: 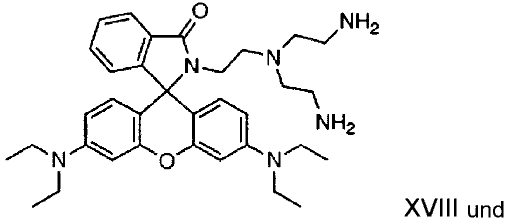
einer Verbindung der Formel XIX: 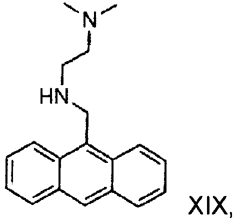
oder Salzen oder Solvaten davon.

5. Kombination nach Anspruch 4, wobei die Kombination weiters eine oder mehrere Verbindungen umfasst, die aus den folgenden ausgewählt sind: 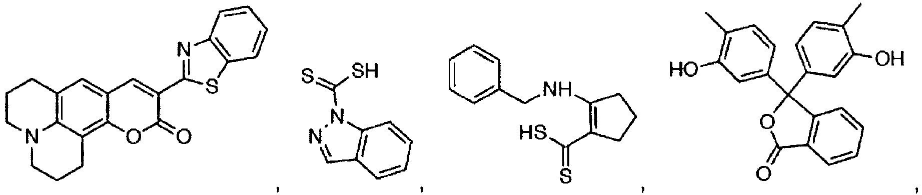 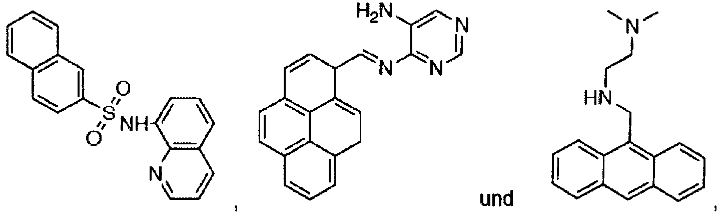 oder Salzen oder Solvaten davon.

6. Kombination nach Anspruch 4 oder Anspruch 5, wobei die Kombination Folgendes umfasst:
- eine Verbindung der Formel I, vorzugsweise la, und
- eine oder mehrere Verbindungen, die aus den Verbindungen der Formeln II-VI, VIII, XII und XIII sowie Salzen und Solvaten davon ausgewählt sind, oder alternativ dazu eine oder mehrere Verbindungen, die aus den Verbindungen der Formeln II-VI, IX, XI, XVIII und XIX sowie Salzen und Solvaten davon ausgewählt sind.

7. Kombination nach einem der Ansprüche 3 bis 6, wobei die Kombination eine Verbindung der Formel I, vorzugsweise la, und eine oder mehrere Verbindungen der Formeln II-VI oder Salze oder Solvate davon umfasst.

8. Verwendung einer Verbindung nach Anspruch 1 oder Anspruch 2 oder einer Kombination nach einem der Ansprüche 3 bis 7 zum Detektieren eines Platin-Komplexes oder einer Kombination von Platin-Komplexen in einer Testprobe.

9. Verfahren zum Detektieren eines Platin-Komplexes in einer Testprobe, wobei das Verfahren Folgendes umfasst:
- das In-Kontakt-Bringen einer Testprobe, von der vermutet wird, dass sie einen Platin-Komplex umfasst, mit einer Verbindung nach Anspruch 1 oder Anspruch 2 oder einer Kombination nach einem der Ansprüche 3 bis 7;
- das Aussetzen der Testprobe gegenüber einer Lichtquelle, die ausreicht, um die Verbindung der Formel I oder die Verbindungen der Kombination anzuregen; und
- das Detektieren von Fluoreszenz von der Verbindung der Formel I oder den Verbindungen der Kombination, wobei das Vorhandensein von Fluoreszenz die Gegenwart des Platin-Komplexes in der Testprobe anzeigt.

10. Verfahren zum Identifizieren eines Platin-Komplexes in einer Testprobe nach Anspruch 9, wobei das Detektionsverfahren für Verbindungen einer Kombination nach einem der Ansprüche 3 bis 7 zusätzlich Folgendes umfasst:
- das Detektieren eines Fluoreszenz-Reaktionsmusters von den Verbindungen der Kombination;
- das Vergleichen des Fluoreszenz-Reaktionsmusters der Testprobe mit einem Bezugsdatensatz in Form von Fluoreszenz-Reaktionsmustern von einer oder mehreren Bezugsproben, die jeweils einen Metallionen-Komplex umfassen, um zu identifizieren, ob es einen Unterschied im Fluoreszenz-Reaktionsmuster zwischen der Testprobe und der einen oder den mehreren Bezugsproben gibt; und
- das Bestimmen der Identität des Platin-Komplexes in der Testprobe, wobei vorzugsweise
- das Fluoreszenz-Reaktionsmuster des Platin-Komplexes in der Testprobe das gleiche ist wie das Fluoreszenz-Reaktionsmuster der Bezugsprobe mit der gleichen Konzentration.

11. Verfahren nach Anspruch 10, wobei das Verfahren weiters das Analysieren des Fluoreszenz-Reaktionsmusters unter Anwendung einer multivariaten Analyse umfasst.

12. Verwendung nach Anspruch 8 oder Verfahren nach einem der Ansprüche 9 bis 11, wobei die Testprobe einen oder mehrere andere Metall-Komplexe als den Platin-Komplex umfasst, wobei der eine oder die mehreren anderen Metall-Komplexe vorzugsweise einen oder mehrere andere Platin-Komplexe umfassen.

13. Verwendung oder Verfahren nach einem der Ansprüche 8 bis 12, wobei die Testprobe eine biologische Probe ist, wobei die biologische Probe vorzugsweise aus Serum, Plasma, Speichel, Tränenflüssigkeit, Harn und Gewebe ausgewählt ist.

14. Verfahren zum Überwachen der Konzentration eines platinhältigen Arzneimittels, vorzugsweise eines Antikrebs-Arzneimittels, bei einem Individuum, das ein Behandlungsregime erhält, welches das platinhältige Arzneimittel umfasst, wobei das Verfahren Folgendes umfasst:
- das Bereitstellen einer biologischen Probe, die von dem Individuum während des Behandlungsregimes erhalten wurde; und
- das Bestimmen der Konzentration des platinhältigen Arzneimittels in der biologischen Probe gemäß einem der Ansprüche 9 bis 13.

15. Set, das eine Verbindung nach Anspruch 1 oder Anspruch 2 oder eine Kombination nach einem der Ansprüche 3 bis 7 umfasst.

## Revendications

1. Composé de formule I : 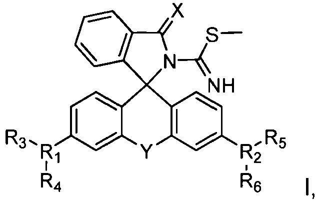 dans laquelle :
X est O ou S ;
Y est choisi parmi O, Si, Se et Ge ;
R₁ et R₂ sont indépendamment N ou O ; et
R₃ à R₆ sont indépendamment absents ou H ou alkyle C₁-C₆, ou sel ou solvate dudit composé.

2. Composé selon la revendication 1, dans lequel le composé a la formule Ia : 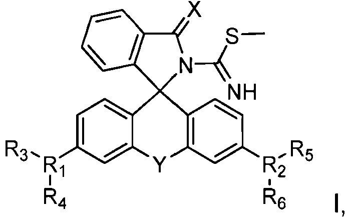 ou sel ou solvate dudit composé.

3. Combinaison comprenant le composé, ou un sel ou solvate de celui-ci, selon la revendication 1 ou 2 et un ou plusieurs composés fluorogènes additionnels.

4. Combinaison selon la revendication 3, dans laquelle le ou les composés fluorogènes additionnels sont choisis parmi :
un composé de formule II : 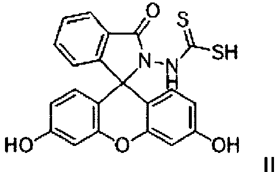
un composé de formule III : 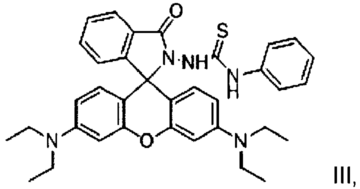
un composé de formule IV : 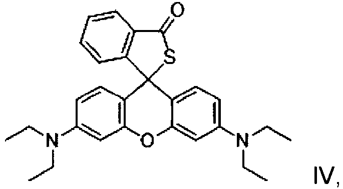
un composé de formule V 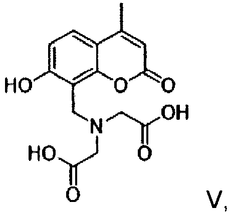
un composé de formule VI : 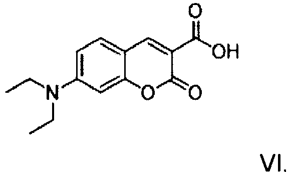
un composé de formule VIII : 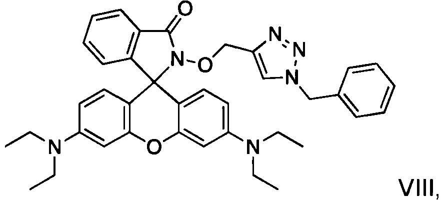
un composé de formule IX : 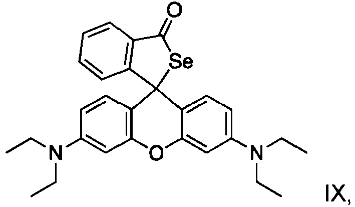
un composé de formule XI : 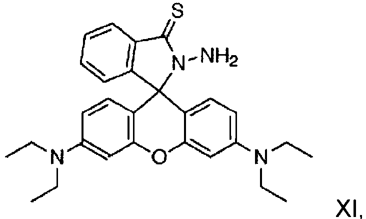
un composé de formule XII : 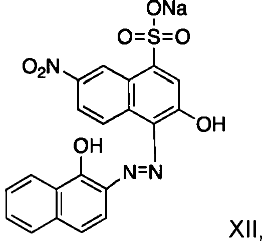
un composé de formule XIII : 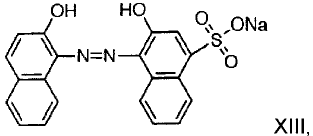
un composé de formule XVIII : 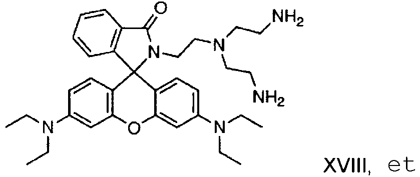
un composé de formule XIX : 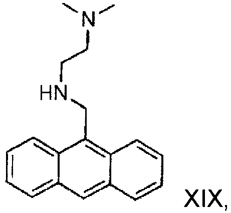
ou des sels ou solvates de ceux-ci.

5. Combinaison selon la revendication 4, dans laquelle la combinaison comprend en outre un ou plusieurs composés choisis parmi : 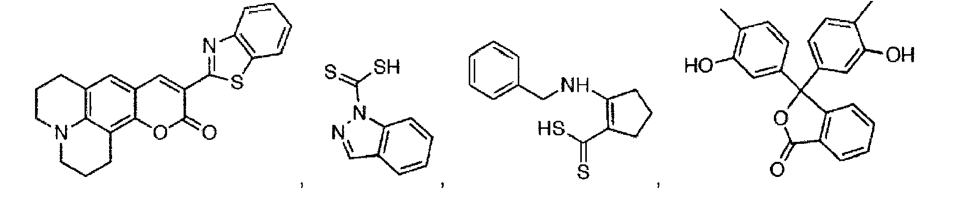 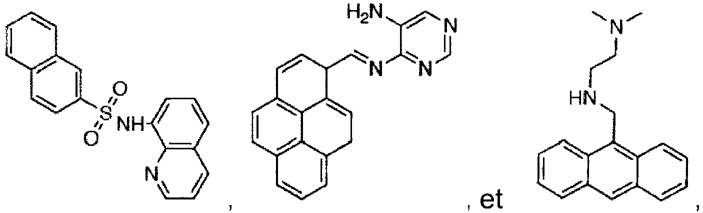 ou des sels ou solvates de ceux-ci.

6. Combinaison selon la revendication 4 ou la revendication 5, dans laquelle la combinaison comprend :
- le composé de formule I, de préférence Ia, et
- un ou plusieurs composé(s) choisi(s) parmi les composés des formules II à VI, VIII, XII et XIII et des sels et solvates de ceux-ci, ou, en variante, un ou plusieurs composés choisis parmi les composés des formules II à VI, IX, XI, XVIII et XIX, et des sels et solvates de ceux-ci.

7. Combinaison selon l'une quelconque des revendications 3 à 6, dans laquelle la combinaison comprend le composé de formule I, de préférence Ia, et un ou plusieurs composés des formules II à VI, ou des sels ou solvates de ceux-ci.

8. Utilisation du composé selon la revendication 1 ou la revendication 2, ou de la combinaison selon l'une quelconque des revendications 3 à 7, pour détecter un complexe de platine ou une combinaison de complexes de platine dans un échantillon à tester.

9. Procédé de détection d'un complexe de platine dans un échantillon à tester, le procédé comprenant les étapes consistant à :
- mettre un échantillon à tester, suspecté de comprendre un complexe de platine, en contact avec le composé selon la revendication 1 ou la revendication 2, ou la combinaison selon l'une quelconque des revendications 3 à 7 ;
- exposer l'échantillon à tester à une source de lumière suffisante pour exciter le composé de formule I ou les composés de la combinaison ; et
- détecter la fluorescence du composé de formule I ou des composés de la combinaison, dans lequel la présence de fluorescence indique la présence du complexe de platine dans l'échantillon à tester.

10. Procédé d'identification d'un complexe de platine dans un échantillon à tester selon la revendication 9, dans lequel le procédé de détection des composés de la combinaison selon l'une quelconque des revendications 3 à 7 comprend en plus les étapes consistant à :
- détecter un profil de réponse de fluorescence des composés de la combinaison ;
- comparer le profil de réponse de fluorescence de l'échantillon à tester à un jeu de données de référence sous la forme de profils de réponse de fluorescence provenant d'un ou plusieurs échantillons de référence comprenant chacun un complexe d'ion métallique afin d'identifier s'il existe une différence dans le profil de réponse de fluorescence entre l'échantillon à tester et le ou les échantillons de référence ; et
- déterminer l'identité du complexe de platine dans l'échantillon à tester, de préférence lorsque
- le profil de réponse de fluorescence du complexe de platine dans l'échantillon à tester est identique au profil de réponse de fluorescence de l'échantillon de référence ayant la même concentration.

11. Procédé selon la revendication 10, dans lequel le procédé comprend en outre l'analyse du profil de réponse de fluorescence au moyen d'une analyse multivariée.

12. Utilisation selon la revendication 8 ou procédé selon l'une quelconque des revendications 9 à 11, dans laquelle/lequel l'échantillon à tester comprend un ou plusieurs complexes métalliques autres que le complexe de platine, de préférence dans laquelle/lequel le ou les autres complexes métalliques comprennent un ou plusieurs autres complexes de platine.

13. Utilisation ou procédé selon l'une quelconque des revendications 8 à 12, dans laquelle/lequel l'échantillon à tester est un échantillon biologique, de préférence dans laquelle/lequel l'échantillon biologique est choisi parmi le sérum, le plasma, la salive, le liquide lacrymal, l'urine et le tissu.

14. Procédé de surveillance de la concentration d'un médicament contenant du platine, de préférence un médicament anticancéreux, chez un sujet recevant un schéma thérapeutique comprenant le médicament contenant du platine, le procédé comprenant les étapes consistant à :
- fournir un échantillon biologique obtenu du sujet pendant le schéma thérapeutique ; et
- déterminer la concentration du médicament contenant du platine dans l'échantillon biologique par le procédé selon l'une quelconque des revendications 9 à 13.

15. Kit comprenant le composé selon la revendication 1 ou la revendication 2 ou la combinaison selon l'une quelconque des revendications 3 à 7.
